# EUROPEAN PATENT SPECIFICATION

(11) **EP 2 532 372 B1**
(45) Date of publication and mention of the grant of the patent: **25.02.2015**
(21) Application number: 11739590.5
(22) Date of filing: 05.01.2011
(51) Int. Cl.: A61L 31/00, A61F 2/82, A61F 2/91, A61F 2/915, A61L 31/14, A61L 31/06

(54) **BIOABSORBABLE STENT**
BIOLOGISCH ABBAUBARER STENT
STENT BIOABSORBABLE

(30) Priority: 02.02.2010 JP 2010021542
(43) Date of publication of application: 12.12.2012
(73) Proprietor: Terumo Kabushiki Kaisha, Tokyo 151-0072 (JP)
(72) Inventor: FUJITA, Yotaro, Fujinomiya-shi Shizuoka 418-0015 (JP); ONISHI, Makoto, Ashigarakami-gun Kanagawa 259-0151 (JP)
(74) Representative: Dossmann, Gérard
(86) International application number: PCT/JP2011/050053
(87) International publication number: WO 2011/096241

(56) References cited:
- EP-A1- 2 243 797
- WO-A1-2008/023038
- WO-A2-2008/036549
- WO-A2-2008/127888
- JP-A- 2005 525 151
- JP-A- 2007 515 249
- JP-A- 2007 526 348
- JP-A- 2009 172 285
- JP-A- 2010 220 746
- US-A1- 2005 137 678
- VOGT F ET AL: "Long-term assessment of a novel biodegradable paclitaxeleluting coronary polylactide stent", EUROPEAN HEART JOURNAL, OXFORD UNIVERSITY PRESS, GB, FR, vol. 25, no. 15, 1 January 2004 (2004-01-01), pages 1330-1340, XP003009053, ISSN: 0195-668X, DOI: 10.1016/J.EHJ.2004.06.010
- LUCIANA LISA LAO ET AL: "Paclitaxel release from single and double-layered poly(DL-lactide- co -glycolide)/poly(L-lactide) film for biodegradable coronary stent application", JOURNAL OF BIOMEDICAL MATERIALS RESEARCH PART A, vol. 87A, no. 1, 1 October 2008 (2008-10-01), pages 1-7, XP055086784, ISSN: 1549-3296, DOI: 10.1002/jbm.a.31706
- PAN P ET AL: "Polymorphism and isomorphism in biodegradable polyesters", PROGRESS IN POLYMER SCIENCE, PERGAMON PRESS, OXFORD, GB, vol. 34, no. 7, 1 July 2009 (2009-07-01), pages 605-640, XP026099487, ISSN: 0079-6700, DOI: 10.1016/J.PROGPOLYMSCI.2009.01.003 [retrieved on 2009-04-10]

## Description

### Technical Field

The present invention relates to a bioabsorbable stent. More particularly, the present invention relates to a bioabsorbable stent for use by insertion and placement in the lumens of a living body such as blood vessel, bile duct, trachea, esophagus, and urethra.

### Background Art

A recent way of coping with stenosis in lumens of a living body, such as blood vessels, particularly the coronary artery, is by the insertion and dilation of a balloon catheter in the stenosis to expand the blood vessel and keep the lumen open.

An example of the foregoing usage of a balloon catheter is illustrated below with reference to the angioplasty to be applied to the ischemic heart disease. Patients of ischemic heart diseases (such as angina pectoris and myocardial infarction) are sharply increasing in number in Japan owing to its westernized eating habit. They undergo percutaneous transluminal coronary angioplasty (PTCA) for the curing of lesion in the coronary artery, and this surgical operation has widely spread. Due to its recent technological development, PTCA is now applied to a large variety of cases, ranging from those in which the lesion is short and the stenosis occurs at one part in the early stage of PTCA to those in which stenosis occurs at more than one part, involving distal eccentric calcification. PTCA is a procedure involving steps of fixing an introducer sheath to a small dissected part of the artery of the patient's leg or arm, inserting a hollow tube called a guide catheter into the blood vessel through the lumen of the introducer sheath, with the help of a guide wire advancing ahead of the guide catheter, placing the guide catheter at the entrance of the coronary artery and then withdrawing the guide wire, inserting another guide wire and a balloon catheter into the lumen of the guide catheter, advancing the balloon catheter to the lesion in the coronary artery of the patient under X-ray radiography, with the guide wire advancing ahead of the balloon catheter, placing the balloon catheter at the lesion, and dilating the balloon at a prescribed pressure for 30 to 60 seconds one to several times. In this way it is possible to expand that part of the blood vessel which has the lesion, thereby increasing the blood flow through the blood vessel. It is reported, however, that the above-mentioned PTCA results in restenosis at a rate of about 30 to 40% as the result of the catheter damaging the wall of the blood vessel, thereby causing the proliferation of the tunica intima which is the curing reaction in the wall of the blood vessel.

One way under study to prevent restenosis is to use such medical devices as stent and atheroma excision catheter. This has been successful to some extent. The stent denotes a tubular medical device to cure diseases caused by stenosis or occlusion in the blood vessel or other lumens. It is so designed as to expand the part of stenosis or occlusion and to be placed there to ensure the lumen. The stent is mostly made of metallic or polymeric material. It is available in various forms, such as a tube of metallic or polymeric material with small pores formed therein and a cylinder braided with wires of metallic material or filaments of polymeric material. The placement of the stent in the blood vessel is intended to prevent or reduce the occurrence of restenosis after PTCA. In fact, however, the placement of the stent by itself is unable to remarkably prevent restenosis.

To address the foregoing problems, there has recently been proposed a stent loading a physiologically active agent, such as immunosuppressive agent and anticancer agent. This stent is designed to release the physiologically active agent over a prolonged period of time at that part of the lumen where the stent is placed, thereby decreasing the possibility of restenosis. An example of such stents is disclosed in EP 0 623 354 A1. It is a stent of tantalum which is coated with a mixture of a substance for curing and a biodegradable polymeric material. Another example is disclosed in Japanese Patent Laid-open No. Hei 9-56807. It is a stent of stainless steel which has thereon a drug layer and a biodegradable polymer layer for eluting drug which are formed one over the other.

The disadvantage of the stents disclosed in EP 0 623 354 A1 or Japanese Patent Laid-open No. Hei 9-56807 is that the stent body is made of metallic material such as stainless steel or tantalum and hence it remains in the living body semipermanently after its placement. This means that the stent body gives a mechanical stress to the wall of the blood vessel, thereby causing chronic inflammation after the decomposition of the biodegradable polymer and the release of the physiologically active agent in the living body. The foregoing is applicable not only to the stents disclosed in EP 0 623 354 A1 or in Japanese Patent Laid-open No. Hei 9-56807 but also to any stent made of metallic material.

In addition, it is reported in Circulation 2002, 2649-2651 that as the result of the polymeric layer remaining semipermanently in the living body, it brings about chronic inflammation and the deterioration of the polymeric layer induces restenosis and intercurrent thrombosis.

In order to address the foregoing problems, there has been disclosed in EP 0 528 039 A1 a technology for forming the stent body with polylactic acid. The advantage of the technology disclosed in EP 0 528 039 A1 is that the polylactic acid constituting the stent body decomposes and the stent body disappears. Consequently, there is no possibility of the chronic inflammation occurring as the result of the stent giving mechanical stress to the wall of the blood vessel after placement in the living body for a long period of time. Thus, the stent mentioned above is less or very little invasive to the patient.

Another stent made of an oriented resorbable material is described in US 2005/137678. Said material is defined as having Young's Modulus in the oriented state greater than Young's modulus of the same resorbable material in an unoriented state.

### Summary of Invention

The above-mentioned stent made of polylactic acid, as disclosed in EP 0 528 039 A1, still has a problem of being poor in radial force because it is made of polylactic acid lacking mechanical strength. With a poor radial force, the stent cannot be placed certainly at the desired position (lesion). Otherwise, the stent shrinks (recoils) inward after placement at the lesion, thereby occluding the lesion again.

The present invention was completed in view of the foregoing. Thus, it is an object of the present invention to provide a bioabsorbable stent that has a high radial force and hence can be placed certainly at the lesion without the possibility of occluding the lesion again after placement.

After their extensive investigation to tackle the foregoing problems, the present inventors found that a stent formed from a bioabsorbable aliphatic polyester and an aromatic compound according to claim 1 exhibits a high radial force which permits it to be placed certainly at the desired position (lesion) in the living body, without the possibility of occluding the lesion again after placement. This finding led to the present invention.

The object of the present invention is achieved by a bioabsorbable stent formed from a mixture composed of a bioabsorbable aliphatic polyester and an aromatic compound according to claim 1.

The bioabsorbable stent according to the present invention has a high radial force and hence it can be certainly placed at the lesion without the possibility of occluding the lesion again after placement at the lesion. Brief Description of Drawings

[Fig. 1]

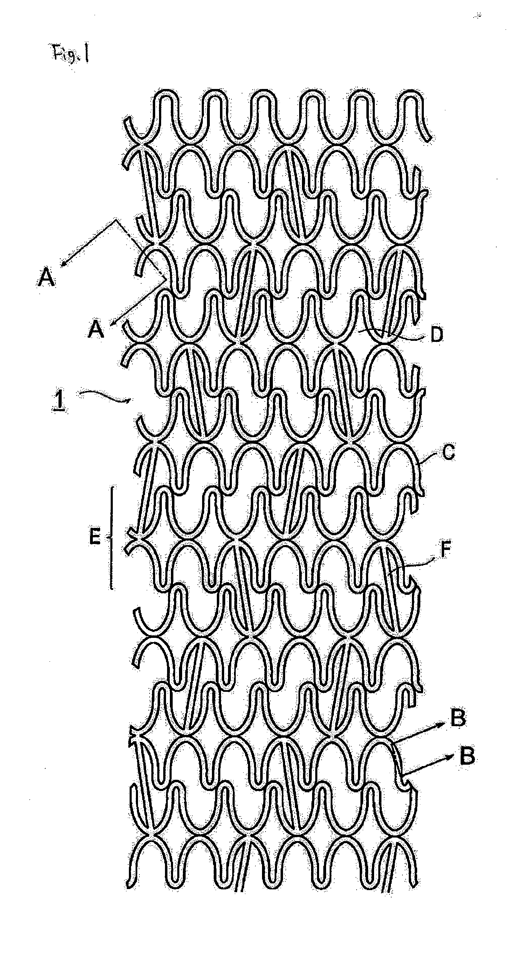

   Fig. 1 is a side view of the stent according to one embodiment of the present invention. In Fig. 1, 1 denotes the stent, C denotes a linear member, D denotes a roughly rhombic element, E denotes an annular unit, and F denotes a connecting member.
[Fig. 2]

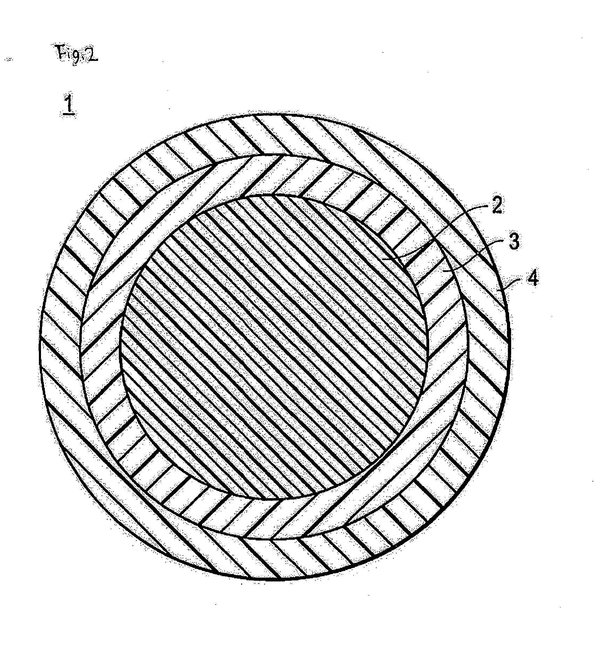

   Fig. 2 is an enlarged cross sectional view taken along the line A-A in Fig. 1. In Fig. 2, 1 denotes the stent, 2 denotes the stent body, 3 denotes a physiologically active agent layer, and 4 denotes a biodegradable polymer layer.
[Fig. 3]

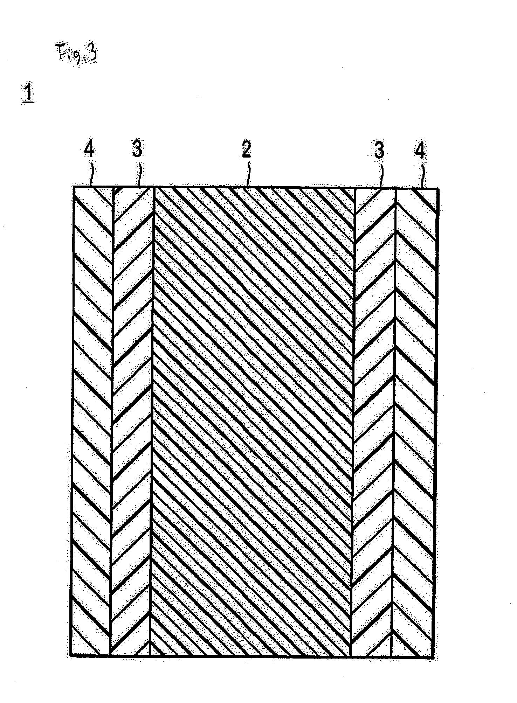

   Fig. 3 is an enlarged longitudinal sectional view taken along the line B-B in Fig. 1. In Fig. 3, 1 denotes the stent, 2 denotes the stent body, 3 denotes a physiologically active agent layer, and 4 denotes a biodegradable polymer layer.
[Fig. 4]

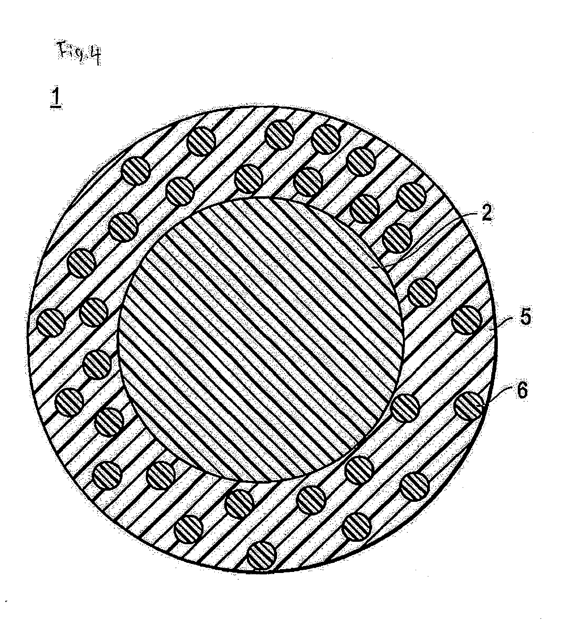

   Fig. 4 is another enlarged cross sectional view taken along the line A-A in Fig. 1. In Fig. 4, 1 denotes the stent, 2 denotes the stent body, 5 denotes a biodegradable polymer layer, and 6 denotes a physiologically active agent layer.
[Fig. 5]

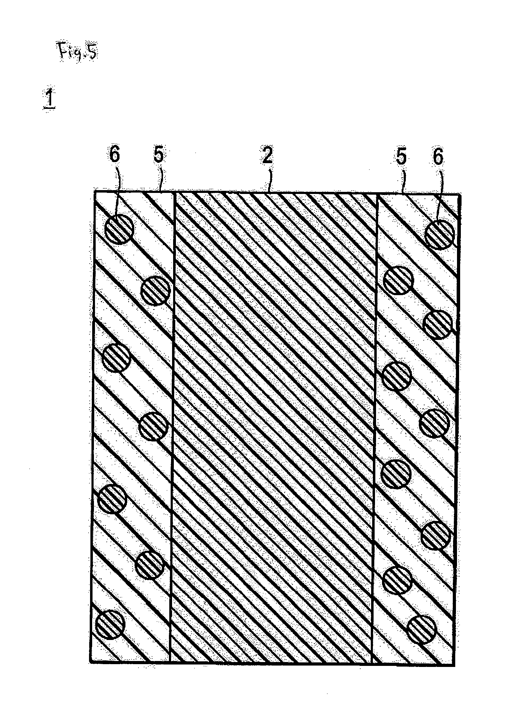

   Fig. 5 is another enlarged longitudinal sectional view taken along the line B-B in Fig. 1. In Fig. 5, 1 denotes the stent, 2 denotes the stent body, 5 denotes a biodegradable polymer, and 6 denotes a physiologically active agent.
[Fig. 6]

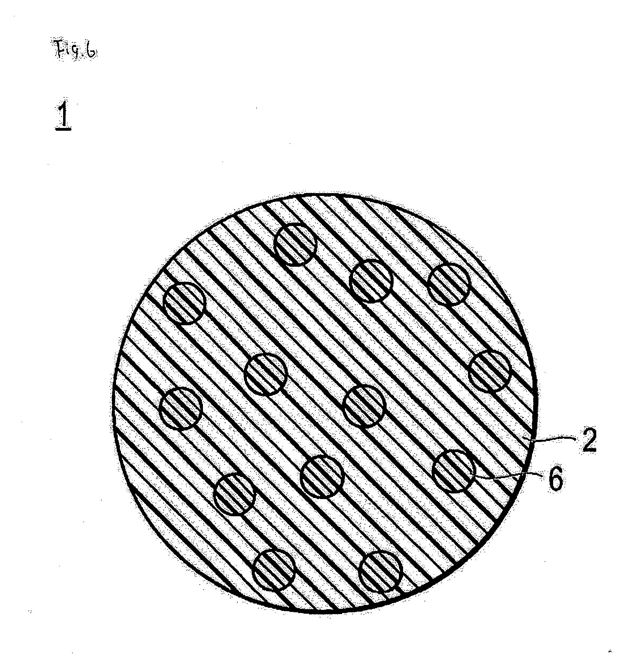

   Fig. 6 is further another enlarged cross sectional view taken along the line A-A in Fig. 1. In Fig. 6, 1 denotes the stent, 2 denotes the stent body, and 6 denotes a physiologically active agent.
[Fig. 7]

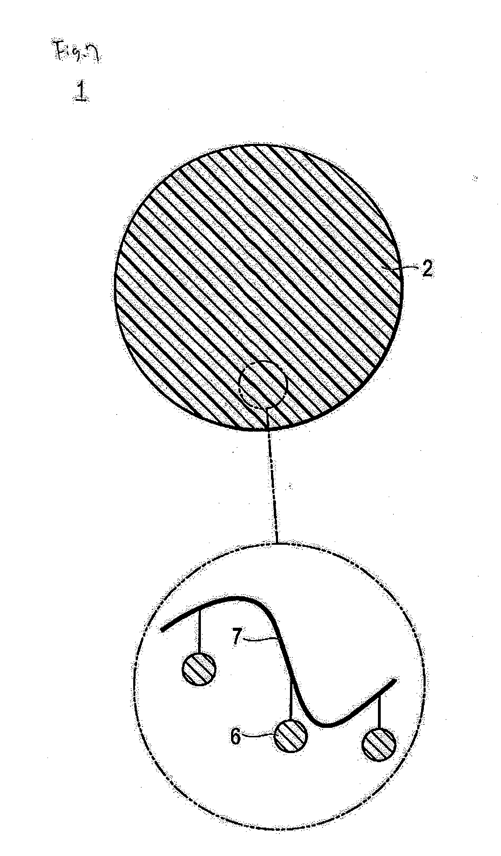

   Fig. 7 is further another enlarged cross sectional view taken along the line A-A in Fig. 1. In Fig. 7, 1 denotes the stent, 2 denotes the stent body, 6 denotes a physiologically active agent, and 7 denotes a bioabsorbable aliphatic polyester.
[Fig. 8]

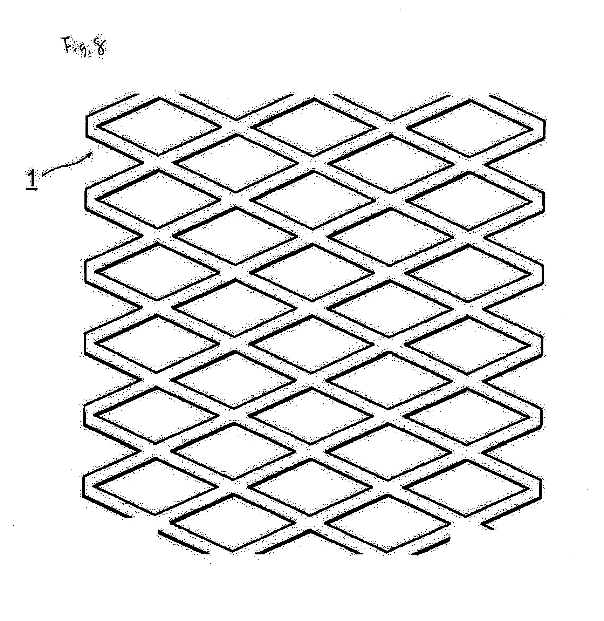

   Fig. 8 is a side view of the stent according to another embodiment of the present invention.

### Mode for Carrying Out the Invention

Provided by the present invention is a bioabsorbable stent (simply referred to as stent hereinafter) which is formed from a mixture composed of a bioabsorbable aliphatic polyester and an aromatic compound according to claim 1. . This aromatic compound causes the molecular chains of the aliphatic polyester to arrange themselves regularly on account of the stacking action of the aromatic rings, with the result that the stent increases in mechanical strength (radial force) while possessing adequate flexibility. Consequently, the stent according to the present invention can be placed certainly at the lesion and can remain there without shrinking (recoiling) inward to prevent occluding of the lesion again. In addition, the stent according to the present invention contains a bioabsorbable aliphatic polyester, which undergoes chemical decomposition, so that the stent is eventually biodegraded and absorbed into the living body. Thus, the stent according to the present invention eliminates the possibility of causing chronic inflammation due to its mechanical stress given to the wall of the blood vessel after placement in the living body for a long period of time. In other words, it is not invasive or very little invasive to the living body. Incidentally, the term "radial force" used in this specification implies the force (rebound) in a radial direction of the blood vessel which the stent exerts on the wall of the blood vessel. To be more specific, it denotes a value which is obtained when a stent, measuring 3 mm in outside diameter and 10 mm long, is compressed by 1 mm at a compression rate of 10 mm/min and its radial force (rebound) is measured by using an autograph (Model AG-IS made by Shimadzu Corporation).

The following is a description of the embodiments of the present invention.

The stent according to the present invention contains a bioabsorbable aliphatic polyester, which undergoes decomposition and absorption in a living body with time. Consequently, it does not stay in a living body for a long period of time and hence it does not give any mechanical stress to the wall of the blood vessel, which eliminates the possibility of causing chronic inflammation. In other words, it is not invasive or very little invasive to a living body. In addition, it may contain physiologically active agents (as described later), which are gradually released with time as the bioabsorbable aliphatic polyester undergoes biodegradation and absorption.

The bioabsorbable aliphatic polyester mentioned above is not specifically restricted, but it should preferably be one which is highly stable in the living body. Its typical examples include the following. Polylactic acid, polyglycolic acid, copolymer of lactic acid and glycolic acid, polycaprolactone, copolymer of lactic acid and caprolactone, copolymer of glycolic acid and caprolactone, polytrimethylene carbonate, copolymer of lactic acid and trimethylene carbonate, copolymer of glycolic acid and trimethylene carbonate, polydioxane, polyethylene succinate, polybutylene succinate, polybutylene succinate-adipate, polyhydroxybutylic acid, and polymalic acid. Preferable among them are polylactic acid, polyglycolic acid, copolymer of lactic acid and glycolic acid, polycaprolactone, copolymer of lactic acid and caprolactone, copolymer of glycolic acid and caprolactone, polytrimethylene carbonate, copolymer of lactic acid and trimethylene carbonate, copolymer of glycolic acid and trimethylene carbonate, polydioxane, polyethylene succinate, polybutylene succinate, and polybutylene succinate-adipate. More preferable among them are polylactic acid, polyglycolic acid, copolymer of lactic acid and glycolic acid, copolymer of lactic acid and trimethylene carbonate, and copolymer of glycolic acid and trimethylene carbonate. They are degradable in a living body and yet they enjoy high medical safety. Incidentally, the above-mentioned bioabsorbable aliphatic polyesters may be used alone or in combination with one another as a mixture. In addition, the aliphatic ester as a constituent of the bioabsorbable aliphatic polyester may contain lactic acid of any optical isomer. In other words, the polylactic acid may include L-polylactic acid, D-polylactic acid, and D,L-polylactic acid. Moreover, the bioabsorbable aliphatic polyester in copolymer form is not specifically restricted in structure. It may be in the form of block copolymer, random copolymer, graft copolymer, or alternating copolymer. Further, the bioabsorbable aliphatic polyester may be obtained commercially or by synthesis. Any known method can be used for synthesis. For example, polylactic acid may be obtained from L-lactic acid or D-lactic acid, whichever necessary, by dehydration and condensation through the lactide method or the direct polymerization method.

The bioabsorbable aliphatic polyester mentioned above is not specifically restricted in weight-average molecular weight so long as it is absorbable in a living body. The weight-average molecular weight is shown below in the order of preference. 10,000 to 3,000,000, 20,000 to 2,000,000, 50,000 to 1,000,000, 60,000 to 500,000, and 80,000 to 300,000. With the foregoing weight-average molecular weight, the bioabsorbable aliphatic polyester exhibits satisfactory biodegradability, bioabsorbability, moldability, and mechanical strength. Incidentally, the "weight-average molecular weight" may be determined by any known method, such as GPC, light scattering method, viscosity measurement method, and mass spectrometry (such as TOFMASS). In this specification, the "weight-average molecular weight" denotes the value determined by using polystyrene, whose molecular weight is known by GPC, as the reference material.

The stent according to the present invention also contains an aromatic compound according to claim 1. The aromatic rings existing in the aromatic compound cause the molecular chains of the aliphatic polyester to regularly arrange by its stacking action. This leads to improvement in the stent's mechanical strength (radial force). Having adequate flexibility, the stent can be placed certainly at the lesion. Once it is placed at the lesion, the stent does not shrink (or recoil) inward to prevent occluding of the lesion again. Moreover, the aromatic compound becomes stable and crystallizes easily as its aromatic rings (such as benzene rings) come close together. This is the reason why the stent according to the present invention exhibits flexibility as well as high radial force (or high mechanical strength), so that it can be placed certainly at the lesion. The stent which has been placed at the lesion does not recoil.

The aromatic compound include the following. 2-hydroxybenzoic acid (salicylic acid), 3-hydroxybenzoic acid, 4-hydroxybenzoic acid, 2,3-dihydroxybenzoic acid, 2,4-dihydroxybenzoic acid, 2,5-dihydroxybenzoic acid, 2,6-dihydroxybenzoic acid, 3,5-dihydroxybenzoic acid, 2-hydroxycinnamic acid, 3-hydroxycinnamic acid, 4-hydroxycinnamic acid, 4-hydroxy-2-methoxycinnamic acid, 4-hydroxy-3-methoxycinnamic acid, 3,4-dihydroxycinnamic acid, mandelic acid, and tyrosine. Incidentally, the foregoing aromatic compound may be an iodized one, which permits the stent to be visible under X-ray radiography. This enables the operator to easily confirm the stent's position with the help of X-rays. The iodized aromatic compound is not specifically restricted; it may be commercially available or may be synthesized by any known method. Preferable among the foregoing aromatic compounds are 2-hydroxybenzoic acid, 3-hydroxybenzoic acid, 4-hydroxybenzoic acid, 2-hydroxycinnamic acid, 3-hydroxycinnamic acid, 4-hydroxycinnamic acid, mandelic acid, and tyrosine, and iodides thereof. More preferable among them are 2-hydroxybenzoic acid, 3-hydroxybenzoic acid, 4-hydroxybenzoic acid, 4-hydroxycinnamic acid, mandelic acid, and tyrosine, and iodides thereof. Incidentally, the foregoing aromatic compounds may be used alone or in combination with one another as a mixture.

The stent according to the present invention is made of the bioabsorbable aliphatic polyester and the aromatic compound both mentioned above. The two components may be mixed in any ratio without specific restrictions so long as the resulting stent exhibits excellent biodegradability and bioabsorbability, a high radial force (mechanical strength), adequate flexibility, and low invasiveness. An adequate mixing ratio of the bioabsorbable aliphatic polyester to the aromatic compound (bioabsorbable aliphatic polyester:aromatic compound by mass) ranges from 100:0.1 to 100:9, preferably from 100:0.5 to 100:8, and more preferably 100:1 to 100:7. Such mixing ratios are desirable for the stent to exhibit excellent biodegradability and bioabsorbability, a high radial force (mechanical strength), adequate flexibility, and low invasiveness.

The stent according to the present invention is not specifically restricted in shape so long as it is strong enough to be stably placed in the lumen of a living body. A preferred shape is a cylinder braided with filaments or a tube having pores in its wall. In addition, the stent according to the present invention may be either of balloon-expandable type or self-expandable type. It should have an adequate size in conformity with the position where it is placed. The preferable outside diameter of the stent before expansion is 1.0 to 5.0 mm, more preferably 1.50 to 4.50 mm, and the preferable length is 5 to 100 mm, more preferably 7 to 50 mm. The stent should have an adequate wall thickness which is not specifically restricted so long as the stent can be placed at the lesion and stent exhibits a radial force necessary to keep the lesion open after placement and ensure the blood flow. The preferable wall thickness is 1 to 1000 µm, more preferably 50 to 300 µm.

It is simply necessary for the stent according to the present invention to contain a mixture of the bioabsorbable aliphatic polyester and the aromatic compound both mentioned above. Therefore, the stent according to the present invention may be formed only from a mixture of the bioabsorbable aliphatic polyester and the aromatic compound or from said mixture incorporated with additional components. Such additional components are not specifically restricted; any ones conventionally used for stents are acceptable. They typically include physiologically active agents and biodegradable polymers.

The physiologically active agents are not specifically restricted and can be selected arbitrarily so long as they produce the effect of preventing restenosis and occlusion after the stent has been placed at the lesion in the lumen. Their typical examples include the following. Anticancer drug, immunosuppresive drug, antibiotic, antirheumatic drug, antithrombotic drug, HMG-CoA (hydroxymethylglutaryl CoA) reductase inhibitor, ACE inhibitor (andiotensin conversion enzyme inhibitor), calcium antagonist, antihyperlipidemic drug, integrin inhibitor, antiallergic drug, antioxidant, GPIIb/IIIa antagonist, retinoid, flavonoid, carotenoid, lipid improver, DNA synthesis inhibitor, tyrosine kinase inhibitor, antiplatelet drug, drug to prevent proliferation of smooth muscle of blood vessel, antiinflammatory drug, tissue-derived biomaterial, interferon, and NO generation promoting agent.

The anticancer drug includes, for example, vincristine, vinblastine, vindesine, irinotecan, pirarubicin, paclitaxel, docetaxel, and methotrexate.

The immunosuppresive drug includes, for example, sirolimus, everolimus, biolimus, tacrolimus, azathioprine, ciclosporin, cyclophosphamide, mycophenolate mofetil, gusperimus, and mizoribine.

The antibiotic includes, for example, mitomycin, adriamycin, doxorubicin, actinomycin, daunorubicin, idarubicin, pirarubicin, aclarubicin, epirubicin, peplomycin, and zinostatin stimalamer.

The antirheumatic drug includes, for example, methotrexate, sodium thiomalate, penicillamine, and lobenzarit.

The antithrombotic drug includes, for example, heparin, aspirin, antithrombin drug, ticlopidine, and hirudin.

The HMG-CoA reductase inhibitor includes, for example, cerivastatin, cerivastatin sodium, atorvastatin, rosuvastatin, pitavastatin, fluvastatin, fluvastatin sodium, simvastatin, lovastatin, and pravastatin.

The ACE inhibitor includes, for example, quinapril, perindopril erbumine, trandolapril, cilazapril, temocapril, delapril, enalapril maleate, lisinopril, and captopril.

The calcium antagonist includes, for example, hifedipine, nilvadipine, diltiazem, benidipine, and nisoldipine.

The antihyperlipidemic drug includes, for example, probucol. The integrin inhibitor includes, for example, AJM300. The antiallergic drug includes, for example, tranilast. The antioxidant includes, for example, catechins, anthocyanin, proanthocyanidin, lycopene, and β-carotene. Preferable among cathechins is epigallocathechin gallate. The GPIIb/IIIa antagonist includes, for example, abciximab.

The retinoid includes, for example, all-trans retinoic acid. The flavonoid includes, for example, epigallocatechin, anthocyanin, and proanthocyanidin. The carotenoid includes, for example, β-carotene and lycopene. The lipid improver includes, for example, eicosapentaenoic acid.

The DNA synthesis inhibitor includes, for example, 5-FU. The tyrosine kinase inhibitor includes, for example, genistein, tyrphostine, and erbstatin. The antiplatelet drug includes, for example, ticlopidine, cilostazol, and clopidogrel. The antiinflammatory drug includes, for example, such steroid as dexamethasone and prednisolone.

The tissue-derived biomaterial includes, for example, EGF (epidermal growth factor), VEGF (vascular endothelial growth factor), HGF (hepatocyte growth factor), PDGF (platelet derived growth factor), and BFGF (basic fibrolast growth factor).

The interferon includes, for example, interferon-γ1a. The NO generation promoting substance includes, for example, L-arginine.

The foregoing physiologically active agents may be used alone or in combination with one another as a mixture. At least one of them should be contained in the bioabsorbable stent for positive prevention of restenosis. Whether one or two or more of the physiologically active agents should be used depends on cases. The physiologically active agents to be contained in the bioabsorbable stent are not restricted in amount and should be selected depending on cases. A preferable amount is 1 to 50 wt%, more preferably 5 to 20 wt%, for the total amount of the bioabsorbable aliphatic polyester and the aromatic compound both mentioned above. This amount is sufficient for positive prevention of restenosis and occulusion.

The biodegradable polymer is a polymer which gradually decomposes after the stent according to the present invention has been placed at the lesion. It is not specifically restricted so long as it has no adverse effect on the living body of human or animal. Those excelling in living body stability are more preferable. Typical examples of the biodegradable polymer include not only the above-mentioned bioabsorbable aliphatic polyester but also at least one polymer selected from poly-α-amino acid, collagen, laminin, heparan sulfate, fibronectin, vitronectin, chondroitin sulfate, hyaluronic acid, and polymer of cinnamic acid or cinnamic acid derivative, and copolymer of the constituent of said polymer with an arbitrary monomer and a mixture of said polymer and copolymer. The term "mixture" used in this specification is a broad concept covering complexes such as polymer alloys. In addition, the biodegradable polymer is not specifically restricted in the weight-average molecular weight, which may range preferably from 10,000 to 1,000,000, more preferably from 20,000 to 500,000, further preferably from 50,000 to 200,000. The "weight-average molecular weight" just mentioned above may be measured by any known method including GPC, light-scattering method, viscosity method, and mass spectrometry (such as TOFMASS). The term "weight -average molecular weight" used in this specification denotes the value determined by GPC that employs polystyrene of known molecular weight as the reference substance. The above-mentioned biodegradable polymers may be used alone or in combination with one another as a mixture.

The foregoing biodegradable polymer is not necessarily essential but may be contained where the bioabsorbable stent needs to have a certain degree of biodegradability. The content of the biodegradable polymer in the bioabsorbable stent is not specifically restricted; it should be properly established according to the patient's graveness, case, and past history in addition to the certain degree of biodegradability. The content of the biodegradable polymer should preferably be 1 to 50 mass%, more preferably 5 to 20 mass%, for the total amount of the bioabsorbable aliphatic polyester and aromatic compound.

The stent according to the present invention can be produced by any known methods, alone or in combination, without specific restrictions. Typical examples of such known methods include blow molding, extrusion molding, injection molding, rotational molding, blow molding, transfer molding, press molding, and solution casting. Preferable among these methods are extrusion molding, blow molding, and injection molding, with blow molding being most desirable.

The stent usually needs sufficient strength in its radial direction (or radial force) so that it withstands compressive force in its radial direction or the structural load which it receives when it supports the wall of the lumen such as blood vessel. The strength in radial direction is associated with the strength in circumferential direction (radial force) of the stent. The stent needs the higher strength in circumferential direction. In addition, the stent should have sufficient flexibility and strength in its lengthwise direction so that it withstands expansion and bending which it experiences during operation and after placement. In other words, the stent needs strength as well as flexibility in its lengthwise direction.

Blow molding among the foregoing molding methods is designed to apply a stress to a mixture of the bioabsorbable aliphatic polyester and the aromatic compound, thereby deforming the mixture in the circumferential (or radial) direction. The applied stress brings about the molecular orientation of the bioabsorbable aliphatic polyester in the direction of stress application, thereby imparting improved mechanical characteristics (radial force) in the circumferential direction (or radial direction). It is to be noted that the "molecular orientation" means the relative orientation of the polymer chains along the longitudinal or circumferential (radial) direction of the polymer chains, in the present invention, the "molecular orientation" is intended for the relative orientation of the polymer chains along the circumference (or radius). Therefor, blow molding causes the bioabsorbable aliphatic polyester constituting the stent according to the present invention to undergo molecular orientation in the circumferential (radial) direction, thereby making the resulting stent improve in mechanical strength (radial force). This suggests that the stent according to the present invention should preferably be formed by blow molding so that the resulting stent is placed certainly at the lesion. Additionally, mechanical failure and shrinking (or recoiling) inward after placement are prevented or reduced.

As mentioned above, it is most desirable to form the stent according to the present invention by blow molding from a mixture of a bioabsorbable aliphatic polyester and an aromatic compound having one or more aromatic rings. The following is a detailed description of the method for producing the stent according to the present invention.

The first step of the production method is to form a parison by a known method such as extrusion molding from a mixture of a bioabsorbable aliphatic polyester and an aromatic compound. The resulting parison is not specifically restricted in size. For example, the outside diameter of the parison should preferably be 0.9 to 2.0 mm, more preferably 1.00 to 1.80 mm in the case of blow molding to the outside diameter of 3.00 mm. The length of the parison should preferably be 5 to 100 mm, more preferably 7 to 50 mm. The wall thickness of the parison should preferably be 100 to 1,000 µm, more preferably 200 to 600 µm.

In the next step, the parison is expanded by introduction of a fluid thereinto under pressure, thereby deforming the parison in its radial direction. In this way the blow molding is completed to form the stent according to the present invention. Incidentally, the foregoing process may be carried out in such a way that the parison is stretched with one end, with the other end thereof fixed, in its axial direction, or the parison is stretched with both ends. Alternatively, the axial stretching may be accomplished before, during, or after (or during and after) expansion in the radial direction.

The blow molding may have an initial step of positioning the parison in a cylindrical member or mold. The mold restricts the deformation of the parison's outside diameter or surface in conformity with the inside diameter of the mold so that the deformation of the parison in its radial direction is controlled. The inside diameter of the mold may be smaller than the desirable diameter of the parison. The deformation of the parison in its radial direction may be controlled by introduction of a fluid at a properly controlled temperature and pressure in place of the mold or in combination with the mold.

The blow molding may be accomplished under any conditions so long as it produces the stent of desired shape. For example, the blow molding temperature should preferably be 40 to 250°C, more preferably 50 to 180°C. The pressure of the fluid to be introduced into the parison during blow molding should preferably be 0.01 to 10.0 MPa, more preferably 0.1 to 8.0 MPa. The duration of fluid introduction should preferably be 10 to 1,200 seconds, more preferably 20 to 600 seconds. Blow molding under the foregoing conditions permits the bioabsorbable aliphatic polyester to undergo adequate molecular orientation in the circumferential direction, thereby imparting adequate mechanical strength (radial force) to the stent. Incidentally, the parison may be heated before, during, or after its deformation. For example, the parison may be heated by introduction of a fluid therein or thereon which is kept at the above-mentioned temperature. This fluid may be the same one as used to apply a pressure into the parison. In addition, the parison may be heated by moving it with a heating element or nozzle juxtaposed thereto. Also, the parison may be heated by the mold. In this case, the mold may be heated by heating any element on or in the mold or an element juxtaposed to the mold. The fluid is an ordinary one, which may be used as such without specific restrictions. Examples of such fluid include air, compressed air, dry nitrogen, oxygen, argon, etc.

The blow molding process may start with a step of closing or sealing one end of the parison. The sealed or closed end may be opened after molding. The sealed parison may be pressurized by introduction of a fluid thereinto. The fluid may be introduced in such a way as to expand the parison in the radial direction. The parison may be permanently deformed by heat-setting by keeping the pressure in it, the tension along its axis, and its temperature higher than its ambient ones. The heat-setting may be achieved by keeping the parison at an adequate temperature, preferably at the desired temperature mentioned above. The duration of time for this purpose may be about one minute to two hours, preferably about two minutes to 10 minutes, although it is not specifically restricted. After the heat-setting, the parison may be cooled and cut in a desired shape, size, and length. After cooling, the deformed parison retains the length and shape conforming to the inside of the mold.

After the blow molding process, the parison is formed into a desired shape, which subsequently undergoes laser etching, chemical etching, laser cutting, or the like according to the desired stent structure. In this way there is obtained the stent according to the present invention. After the blow molding process, the pores may be formed by coating the surface of the parison with a perforated pattern and removing those parts except for the perforated pattern with the help of a laser beam or a chemical solution. The laser cutting may be accomplished by making pores in the surface of the tubular product with the help of a laser beam which is scanned according to a pattern information stored in a computer.

The stent according to the present invention, which is produced by the foregoing process, excels in mechanical strength with adequate flexibility and high radial force. In addition, the stent according to the present invention, which contains a bioabsorbable aliphatic polyester, is eventually decomposed in the living body and absorbed into the living body. Owing to its high radial force, the stent according to the present invention can be placed certainly at the desired position (lesion), and it prevents occluding of the lesion again without shrinking (recoiling) inward once it is placed at the lesion. After fulfilling its function to prevent acute vascular occlusion or restenosis as a stent, the stent is decomposed and absorbed in the living body. This leads to a limited possibility of the stent causing restenosis and complication of thrombosis in the late phase. As the result, the stent according to the present invention prevents chronic inflammation caused by mechanical stress which any conventional stent gives to the wall of the blood vessel after its placement for a long period of time. In other words, the stent according to the present invention is no invasive or very little invasive to the patient.

The structure of the stent according to the present invention will be described below in more detail with reference to one embodiment illustrated in the accompanying drawings. The following description is not intended to restrict the structure of the stent according to the present invention as a matter of course.

Fig. 1 is a side view of the stent according to one embodiment of the present invention. Figs. 2, 4, 6, and 7 are enlarged cross sectional views each taken along the line A-A in Fig. 1. Figs. 3 and 5 are enlarged longitudinal sectional views each taken along the line B-B in Fig. 1. The stent according to the present invention is not limited to the one shown in Fig. 1, but it may be the one with lattice structure as shown in Fig. 8.

The following is a detailed description of elements constituting the stent 1 shown in Fig. 1.

The stent 1 (or the stent body 2) is a cylindrical body having open ends and extending in its axial direction. The wall of the cylindrical body has a large number of cut openings passing through it. The cut openings deform so that the cylindrical body expands and shrinks in its radial direction. Thus the stent maintains its shape after it is placed in the lumen of a living body such as blood vessel and bile duct.

The stent 1 (or the stent body 2) according to the embodiment shown in Fig. 1 is a bioabsorbable stent made of a mixture composed of a bioabsorbable aliphatic polyester of the present invention and an aromatic compound having one or more aromatic rings as mentioned above. It is composed of a roughly rhombic element D with a cut opening therein, as a basic unit. A plurality of roughly rhombic elements continuously arranged in the circumferential direction constitute the annular unit E. Each annular unit E is joined to its adjacent ones through linear connecting members F. In this way the annular units E in plural number are continuously arranged in the axial direction, with a portion of them joined together. The stent 1 (or the stent body 2) constructed as mentioned above constitutes a cylindrical body which has open ends and extends in its axial direction. In addition, the wall of the cylindrical body has roughly rhombic cut openings, and the cylindrical body expands and shrinks in its axial direction as the cut openings deform.

The stent according to the present invention is not restricted to the structure shown in Fig. 1. The structure is based on the idea that the stent is a cylindrical body axially extending between open ends which has a large number of cut openings in its wall and the cylindrical body expands and shrinks in its radial direction as these cut openings deform. Therefore, this idea embraces a coil-like structure. Moreover, the elastic thin members constituting the stent (or stent body) may have any sectional shape, including rectangle, circle, ellipsoid, polygon, etc.

According to the present invention, the stent 1 shown in Fig. 2 is constructed such that the stent body 2 carries thereon a layer that releases a physiologically active agent, said layer being composed of a physiologically active agent and a biodegradable polymer. Moreover, in Fig. 2, the layer that releases a physiologically active agent is composed of the physiologically active agent layer 3, which is in contact with the surface of the stent body 2, and the biodegradable polymer layer 4 which entirely covers the physiologically active agent layer 3.

Moreover, according to the present invention, the stent 1 shown in Fig. 4 is constructed such that the stent body 2 carries thereon a layer that releases a physiologically active agent, said layer being composed of a physiologically active agent and a biodegradable polymer. In this case, the layer that releases a physiologically active agent is composed of a physiologically active agent 6 and a biodegradable polymer 5 which are mixed together.

According to the present invention, the stent 1 shown in Fig. 6 is characterized in that the stent body 2 contains a physiologically active agent 6 dispersed or embedded therein. In this case, too, the stent body 2 may carry thereon a layer (not shown) that releases a physiologically active agent, said layer being composed of a physiologically active agent and a biodegradable polymer.

According to the present invention, the stent 1 shown in Fig. 7 is constructed such that the stent body 2 carries a physiologically active agent 6 chemically bonding thereto. The inset in Fig. 7 is an enlarged view of the stent 1 which shows that the bioabsorbable aliphatic polyester 7 constituting the stent body 2 has a physiologically active agent 6 chemically bonding directly thereto. In other words, the bioabsorbable aliphatic polyester 7 has the physiologically active agent in its side chains, which constitutes the so-called prodrug structure. In this case, too, the stent body 2 may additionally have a layer (not shown) which releases a physiologically active agent,said layer being composed of a physiologically active agent and a biodegradable polymer, as shown in Figs. 2 and 4.

The stent 1 according to the present invention, which is shown in Fig. 3, is the same one as shown in Fig. 2. Fig. 3 is a longitudinal sectional view taken along the line B-B in Fig. 1. The stent body 2 is composed of linear members C constituting the bioabsorbable stent of the present invention; each of the members C carries thereon a layer that releases a physiologically active agent (the physiologically active agent 3 layer and the biodegradable polymer layer 4). Incidentally, the layer that releases a physiologically active agent may cover the stent body 2 entirely or partly. The entire covering is schematically shown in Fig. 3.

The stent 1 according to the present invention, which is shown in Fig. 5, is the same one as shown in Fig. 4. Fig. 5 is a longitudinal sectional view taken along the line B-B in Fig. 1. The stent body 2 is composed of linear members C, each of which carries thereon entirely a layer that releases a physiologically active agent (a layer formed from a mixture of a physiologically active agent 6 and a biodegradable polymer 5). Incidentally, the layer that releases a physiologically active agent may cover the stent body 2 entirely or partly. The entire covering of the entire surface of the linear members C formed of bioabsorbable stent of the present invention constituting the stent body 2 is schematically shown in Fig. 5.

The layer that releases a physiologically active agent should preferably be formed at least on that part of the linear member C which comes into direct contact with the tissue of a living body. This causes the physiologically active agent released from the layer to be absorbed directly into the tissue of a living body without being dissolved in the body fluid (blood). The physiologically active agent locally administered in this way exhibits its physiological action more effectively.

In the case where the stent body 2 contains a physiologically active agent or the stent 1 has a physiologically active agent layer or a layer that releases a physiologically active agent, the stent 1 releases the physiologically active agent after it has been placed at the lesion in a living body, thereby preventing restenosis. At the same time, the bioabsorbable aliphatic polyester and the biodegradable polymer are completely decomposed in the living body.

The stent according to the present invention does not require that the stent body 2 have the physiologically active agent layer 3 or the biodegradable polymer layer 4 as mentioned above. The stent body 2 may have thereon the physiologically active agent layer 3 or the biodegradable polymer layer 4. In the latter case, the physiologically active agent layer 3 should have a thickness not harmful to the performance of the stent body 2, such as easy delivery to the lesion and low stimulus to the blood vessel. A preferable thickness should be 1 to 100 µm, more preferably 1 to 50 µm, and most preferable 1 to 20 µm, so that the physiologically active agent fully produces its effect. Likewise, the biodegradable polymer layer 4 should also has an adequate thickness not harmful to the performance of the stent body 2, such as easy delivery to the lesion and low stimulus to the blood vessel as with the physiologically active agent layer 3. A preferable thickness should be 1 to 75 µm, more preferably 1 to 25 µm, and most preferable 1 to 10 µm. The physiologically active agent and biodegradable polymer to be used for the foregoing object are not specifically restricted, but any known ones, such as mentioned above, may be equally acceptable.

Any method may be employed without specific restrictions to form the physiologically active agent layer 3 on the surface of the stent body 2 pertaining to the present invention. It is exemplified below. A method including melting a physiologically active agent and applying the resulting melt onto the surface of the stent body 2. A method including dissolving a physiologically active agent in a solvent and dipping the stent body 2 in the resulting solution, followed by solvent removal by evaporation or the like. A method including spraying the foregoing solution onto the stent body 2, followed by solvent removal by evaporation or the like.

The dipping and spraying methods which employ a solution dissolving only a physiologically active agent in a solvent are simple and preferable in the case where the physiologically active agent can be dissolved in a solvent which makes the surface of the stent body 2 highly wettable.

The physiologically active agent layer 3 should preferably be coated with the biodegradable polymer layer 4. More than one kind of biodegradable polymer may be used in the polymer layer.

Any method may be employed without specific restrictions to form the biodegradable polymer layer 4. It is exemplified below. A method including melting a biodegradable polymer and applying the resulting melt onto the physiologically active agent layer 3 which has been formed on the surface of the stent body 2 as mentioned above. A method including dissolving a biodegradable polymer in a solvent and dipping the stent body 2 which has the physiologically active agent layer 3 formed thereon in the resulting solution, followed by solvent removal by evaporation or the like. A method including spraying the foregoing solution onto the stent body 2 which has the physiologically active agent layer 3 formed thereon, followed by solvent removal by evaporation or the like.

The dipping and spraying methods which employ a solution dissolving only a biodegradable polymer in a solvent are simple and preferable in the case where the biodegradable polymer can be dissolved in a solvent which makes the surface of the stent body 2 which has the physiologically active agent layer 3 formed thereon highly wettable.

The stent body 2 of the present invention may have on the surface thereof the layer of biodegradable polymer 5 which contains the physiologically active agent 6 dispersed or embedded therein.

The stent according to the present invention may be constructed such that the stent body 2 has thereon a layer of biodegradable polymer containing a physiologically active agent dispersed or embedded therein, that is, a layer of mixture of a biodegradable polymer and a physiologically active agent. In this case, the mixing ratio (mass part) of the biodegradable polymer to the physiologically active agent should be from 99:1 to 1:99, preferably from 90:10 to 10:90, more preferably from 70:30 to 30:70.

According to the present invention, the layer of biodegradable polymer 5 containing the physiologically active agent 6 dispersed therein (or mixed therewith) should have an adequate thickness not harmful to the performance of the stent body 2, such as easy delivery to the lesion and low stimulus to the blood vessel. A preferable thickness should be 1 to 100 µm, more preferably 1 to 50 µm, and most preferably 1 to 20 µm such that the physiologically active agent fully produces its effect.

Any method may be employed without specific restrictions to form the layer of biodegradable polymer 5 containing the physiologically active agent 6 dispersed or embedded therein on the surface of the stent body 2 pertaining to the present invention. It is exemplified below. A method including melting a biodegradable polymer and a physiologically active agent and applying the resulting melt onto the surface of the stent body 2. A method including dissolving a biodegradable polymer and a physiologically active agent in a solvent and dipping the stent body 2 in the resulting solution, followed by solvent removal by evaporation or the like. A method including spraying the foregoing solution onto the stent body 2, followed by solvent removal by evaporation or the like.

The dipping and spraying methods which employ a solution dissolving a biodegradable polymer and a physiologically active agent in a solvent are simple and preferable in the case where the biodegradable polymer and the physiologically active agent can be dissolved in a solvent which makes the surface of the stent body 2 highly wettable.

According to the present invention, the layer that releases a physiologically active agent is formed from a composition containing a physiologically active agent and a biodegradable polymer, and it is the physiologically active agent layer 3, the biodegradable polymer layer 4, or the layer of biodegradable polymer 5 containing the physiologically active agent 6 dispersed therein. The layer that releases a physiologically active agent does not always need to cover the entire surface of the linear members constituting the stent body; it is only necessary for the layer that releases a physiologically active agent to cover at least a portion of the surface of the linear elements C constituting the stent body.

It is desirable that the layer that releases a physiologically active agent according to the present invention should cover 1 to 100%, preferably 50 to 100%, of the entire surface area of the stent body.

Also, the stent body 2 according to the present invention may contain the physiologically active agent 6 dispersed or embedded therein. The stent body 2 of such structure may be produced by any method. A desirable simple method includes incorporating a physiologically active agent into the bioabsorbable aliphatic polyester and the aromatic compound at the time of melt molding. There is another method that includes mixing a physiologically active agent with a mixture of the bioabsorbable aliphatic polyester and the aromatic compound. The resulting mixture is biodegradable as well as capable of releasing the biological physiologically active agent. Thus the biological physiologically active agent suppresses inflammation that results from biodegradation of the stent body.

The physiologically active agent 3 dissolves and diffuses into the biodegradable polymer layer 4 or the biodegradable polymer layer 4 or 5 decomposes in the living body, so that the physiologically active agent 3 or 6 is entirely released in the living body. At the same time, the stent body 2 decomposes in the living body. In this way all the constituents of the stent disappear eventually.

The stent 1 according to the present invention which is produced as mentioned above may be expanded in any way without specific restrictions as in the case of ordinary stents. In case of self-expandable type, it will expand in its radial direction by its own restoring force when it is released from the force that keeps the stent folded up small. In the case of balloon-expandable type, the stent is expanded in its radial direction by external force applied from the inside of the stent.

### Examples

In order to demonstrate its effect, the present invention will be described in more detail with reference to the following Examples and Comparative Examples, which are not intended to restrict the technical scope thereof.

### Example 1

A mixture was prepared by dry blending from 100 pbw of polylactic acid (having a weight-average molecular weight of 123,000, made by DURECT Corporation) and 1 pbw of mandelic acid (made by Sigma-Aldrich Corporation). The resulting mixture was formed into a tube, measuring 1.38 mm in outside diameter and 0.45 mm in inside diameter, by extrusion molding (with Labo Plastomill made by Toyo Seiki Seisaku-Sho, Ltd.). The resulting tube underwent blow molding by introduction of pressurized dry nitrogen at 90°C and 0.5 MPa for 120 seconds. Thus there was obtained a blown tube measuring 3.00 mm in outside diameter and 2.70 mm in inside diameter. The thus obtained blown tube was fabricated by means of ArF excimer laser (193 nm) to give the stent of the structure as shown in Fig. 8.

The stent of 3.00 mm in outside diameter and cut to a length of 10 mm was tested for radial force (rebound) exerted by it after compression by 1 mm, by using an autograph (Model AG-IS, made by Shimadzu Corporation). It was found that the radial force (rebound) was 139 gf.

The same procedure as mentioned above was repeated to produce the stent of the structure as shown in Fig. 1. It was found that this stent has a radial force (rebound) of 164 gf.

### Example 2

A mixture was prepared by dry blending from 100 pbw of polylactic acid (made by DURECT Corporation, the same one as used in Example 1) and 1 pbw of 4-hydroxycinnamic acid (made by Tokyo Chemical Industry Co., Ltd.). The resulting mixture was formed into a tube, measuring 1.38 mm in outside diameter and 0.45 mm in inside diameter, by extrusion molding (with Labo Plastomill made by Toyo Seiki Seisaku-Sho, Ltd.). The resulting tube underwent blow molding by introduction of pressurized dry nitrogen at 80°C and 0.5 MPa for 120 seconds. Thus there was obtained a blown tube measuring 3.00 mm in outside diameter and 2.70 mm in inside diameter. The thus obtained blown tube was fabricated by means of ArF excimer laser (193 nm) to give the stent of the structure as shown in Fig. 8.

The stent of 3.00 mm in outside diameter and cut to a length of 10 mm was tested for radial force (rebound) exerted by it after compression by 1 mm in the same way as in Example 1. It was found that the radial force (rebound) was 131 gf.

The same procedure as mentioned above was repeated to produce the stent of the structure as shown in Fig. 1. It was found that this stent has a radial force (rebound) of 155 gf.

### Example 3

A mixture was prepared by dry blending from 100 pbw of polylactic acid (made by DURECT Corporation, the same one as used in Example 1) and 1 pbw of 4-hydroxybenzoic acid (made by Sigma-Aldrich Corporation). The resulting mixture was formed into a tube, measuring 1.38 mm in outside diameter and 0.45 mm in inside diameter, by extrusion molding (with Labo Plastomill made by Toyo Seiki Seisaku-Sho, Ltd.). The resulting tube underwent blow molding by introduction of pressurized dry nitrogen at 85°C and 0.5 MPa for 120 seconds. Thus there was obtained a blown tube measuring 3.00 mm in outside diameter and 2.70 mm in inside diameter. The thus obtained blown tube was fabricated by means of ArF excimer laser (193 nm) to give the stent of the structure as shown in Fig. 8.

The stent of 3.00 mm in outside diameter and cut to a length of 10 mm was tested for radial force (rebound) exerted by it after compression by 1 mm in the same way as in Example 1. It was found that the radial force (rebound) was 150 gf.

The same procedure as mentioned above was repeated to produce the stent of the structure as shown in Fig, 1. It was found that this stent has a radial force (rebound) of 173 gf.

### Example 4

A mixture was prepared by dry blending from 100 pbw of polylactic acid (made by DURECT Corporation, the same one as used in Example 1) and 1 pbw of 3-iodo-L-tyrosine (made by Tokyo Chemical Industry Co., Ltd.). The resulting mixture was formed into a tube, measuring 1.38 mm in outside diameter and 0.45 mm in inside diameter, by extrusion molding (with Labo Plastomill made by Toyo Seiki Seisaku-Sho, Ltd.). The resulting tube underwent blow molding by introduction of pressurized dry nitrogen at 90°C and 0.5 MPa for 120 seconds. Thus there was obtained a blown tube measuring 3.00 mm in outside diameter and 2.70 mm in inside diameter. The thus obtained blown tube was fabricated by means of ArF excimer laser (193 nm) to give the stent of the structure as shown in Fig. 8.

The stent of 3.00 mm in outside diameter and cut to a length of 10 mm was tested for radial force (rebound) exerted by it after compression by 1 mm in the same way as in Example 1. It was found that the radial force (rebound) was 159 gf. This stent was visible under X-ray radiography.

The same procedure as mentioned above was repeated to produce the stent of the structure as shown in Fig. 1. It was found that this stent has a radial force (rebound) of 183 gf. This stent was visible under X-ray radiography.

### Example 5

A mixture was prepared by dry blending from 100 pbw of polylactic acid (made by DURECT Corporation, the same one as used in Example 1) and 1 pbw of 3,5-diiodosalicylic acid (made by Sigma-Aldrich Corporation). The resulting mixture was formed into a tube, measuring 1.38 mm in outside diameter and 0.45 mm in inside diameter, by extrusion molding (with Labo Plastomill made by Toyo Seiki Seisaku-Sho, Ltd.). The resulting tube underwent blow molding by introduction of pressurized dry nitrogen at 90°C and 0.5 MPa for 120 seconds. Thus there was obtained a blown tube measuring 3.00 mm in outside diameter and 2.70 mm in inside diameter. The thus obtained blown tube was fabricated by means of ArF excimer laser (193 nm) to give the stent of the structure as shown in Fig. 8.

The stent of 3.00 mm in outside diameter and cut to a length of 10 mm was tested for radial force (rebound) exerted by it after compression by 1 mm in the same way as in Example 1. It was found that the radial force (rebound) was 150 gf. This stent was visible under X-ray radiography.

The same procedure as mentioned above was repeated to produce the stent of the structure as shown in Fig. 1. It was found that this stent has a radial force (rebound) of 163 gf. This stent was visible under X-ray radiography.

### Example 6

A mixture was prepared by dry blending from 100 pbw of polylactic acid (made by DURECT Corporation, the same one as used in Example 1) and 7 pbw of mandelic acid (made by Sigma-Aldrich Corporation). The resulting mixture was formed into a tube, measuring 1.38 mm in outside diameter and 0.45 mm in inside diameter, by extrusion molding (with Labo Plastomill made by Toyo Seiki Seisaku-Sho, Ltd.). The resulting tube underwent blow molding by introduction of pressurized dry nitrogen at 90°C and 0.5 MPa for 120 seconds. Thus there was obtained a blown tube measuring 3.00 mm in outside diameter and 2.70 mm in inside diameter. The thus obtained blown tube was fabricated by means of ArF excimer laser (193 nm) to give the stent of the structure as shown in Fig. 8.

The stent of 3.00 mm in outside diameter and cut to a length of 10 mm was tested for radial force (rebound) exerted by it after compression by 1 mm in the same way as in Example 1. It was found that the radial force (rebound) was 139 gf.

The same procedure as mentioned above was repeated to produce the stent of the structure as shown in Fig. 1. It was found that this stent has a radial force (rebound) of 164 gf.

### Example 7

A mixture was prepared by dry blending from 100 pbw of polylactic acid (made by DURECT Corporation, the same one as used in Example 1) and 7 pbw of 4-hydroxycinnamic acid (made by Tokyo Chemical Industry Co., Ltd.). The resulting mixture was formed into a tube, measuring 1.38 mm in outside diameter and 0.45 mm in inside diameter, by extrusion molding (with Labo Plastomill made by Toyo Seiki Seisaku-Sho, Ltd.). The resulting tube underwent blow molding by introduction of pressurized dry nitrogen at 80°C and 0.5 MPa for 120 seconds. Thus there was obtained a blown tube measuring 3.00 mm in outside diameter and 2.70 mm in inside diameter. The thus obtained blown tube was fabricated by means of ArF excimer laser (193 nm) to give the stent of the structure as shown in Fig. 8.

The stent of 3.00 mm in outside diameter and cut to a length of 10 mm was tested for radial force (rebound) exerted by it after compression by 1 mm in the same way as in Example 1. It was found that the radial force (rebound) was 131 gf.

The same procedure as mentioned above was repeated to produce the stent of the structure as shown in Fig. 1. It was found that this stent has a radial force (rebound) of 155 gf.

### Example 8

A mixture was prepared by dry blending from 100 pbw of polylactic acid (made by DURECT Corporation, the same one as used in Example 1) and 7 pbw of 4-hydroxybenzoic acid (made by Sigma-Aldrich Corporation). The resulting mixture was formed into a tube, measuring 1.38 mm in outside diameter and 0.45 mm in inside diameter, by extrusion molding (with Labo Plastomill made by Toyo Seiki Seisaku-Sho, Ltd.). The resulting tube underwent blow molding by introduction of pressurized dry nitrogen at 85°C and 0.5 MPa for 120 seconds. Thus there was obtained a blown tube measuring 3.00 mm in outside diameter and 2.70 mm in inside diameter. The thus obtained blown tube was fabricated by means of ArF excimer laser (193 nm) to give the stent of the structure as shown in Fig. 8.

The stent of 3.00 mm in outside diameter and cut to a length of 10 mm was tested for radial force (rebound) exerted by it after compression by 1 mm in the same way as in Example 1. It was found that the radial force (rebound) was 150 gf.

The same procedure as mentioned above was repeated to produce the stent of the structure as shown in Fig. 1. It was found that this stent has a radial force (rebound) of 173 gf.

### Example 9

A mixture was prepared by dry blending from 100 pbw of polylactic acid (made by DURECT Corporation, the same one as used in Example 1) and 7 pbw of 3-iodo-L-tyrosine (made by Tokyo Chemical Industry Co., Ltd.). The resulting mixture was formed into a tube, measuring 1.38 mm in outside diameter and 0.45 mm in inside diameter, by extrusion molding (with Labo Plastomill made by Toyo Seiki Seisaku-Sho, Ltd.). The resulting tube underwent blow molding by introduction of pressurized dry nitrogen at 90°C and 0.5 MPa for 120 seconds. Thus there was obtained a blown tube measuring 3.00 mm in outside diameter and 2.70 mm in inside diameter. The thus obtained blown tube was fabricated by means of ArF excimer laser (193 nm) to give the stent of the structure as shown in Fig. 8.

The stent of 3.00 mm in outside diameter and cut to a length of 10 mm was tested for radial force (rebound) exerted by it after compression by 1 mm in the same way as in Example 1. It was found that the radial force (rebound) was 159 gf. This stent was visible under X-ray radiography.

The same procedure as mentioned above was repeated to produce the stent of the structure as shown in Fig. 1. It was found that this stent has a radial force (rebound) of 183 gf. This stent was visible under X-ray radiography.

### Example 10

A mixture was prepared by dry blending from 100 pbw of polylactic acid (made by DURECT Corporation, the same one as used in Example 1) and 7 pbw of 3,5-diiodo-salicylic acid (made by Sigma-Aldrich Corporation). The resulting mixture was formed into a tube, measuring 1.38 mm in outside diameter and 0.45 mm in inside diameter, by extrusion molding (with Labo Plastomill made by Toyo Seiki Seisaku-Sho, Ltd.). The resulting tube underwent blow molding by introduction of pressurized dry nitrogen at 90°C and 0.5 MPa for 120 seconds. Thus there was obtained a blown tube measuring 3.00 mm in outside diameter and 2.70 mm in inside diameter. The thus obtained blown tube was fabricated by means of ArF excimer laser (193 nm) to give the stent of the structure as shown in Fig. 8.

The stent of 3.00 mm in outside diameter and cut to a length of 10 mm was tested for radial force (rebound) exerted by it after compression by 1 mm in the same way as in Example 1. It was found that the radial force (rebound) was 150 gf. This stent was visible under X-ray radiography.

The same procedure as mentioned above was repeated to produce the stent of the structure as shown in Fig. 1. It was found that this stent has a radial force (rebound) of 163 gf. This stent was visible under X-ray radiography.

### Example 11

A mixture was prepared by dry blending from 100 pbw of lactic acid-trimethylene carbonate copolymer having a weight-average molecular weight of 100,000 (made by Taki Chemical Co., Ltd.) and 1 pbw of mandelic acid (made by Sigma-Aldrich Corporation). The resulting mixture was formed into a tube, measuring 1.38 mm in outside diameter and 0.45 mm in inside diameter, by extrusion molding (with Labo Plastomill made by Toyo Seiki Seisaku-Sho, Ltd.). The resulting tube underwent blow molding by introduction of pressurized dry nitrogen at 60°C and 1.0 MPa for 120 seconds. Thus there was obtained a blown tube measuring 3.00 mm in outside diameter and 2.70 mm in inside diameter. The thus obtained blown tube was fabricated by means of ArF excimer laser (193 nm) to give the stent of the structure as shown in Fig. 8.

The stent of 3.00 mm in outside diameter and cut to a length of 10 mm was tested for radial force (rebound) exerted by it after compression by 1 mm in the same way as in Example 1. It was found that the radial force (rebound) was 131 gf.

The same procedure as mentioned above was repeated to produce the stent of the structure as shown in Fig. 1. It was found that this stent has a radial force rebound) of 158 gf.

### Example 12

A mixture was prepared by dry blending from 100 pbw of lactic acid-trimethylene carbonate copolymer (made by Taki Chemical Co., Ltd., the same one as used in Example 11) and 1 pbw of 4-hydroxycinnamic acid (made by Tokyo Chemical Industry Co., Ltd.). The resulting mixture was formed into a tube, measuring 1.38 mm in outside diameter and 0.45 mm in inside diameter, by extrusion molding (with Labo Plastomill made by Toyo Seiki Seisaku-Sho, Ltd.). The resulting tube underwent blow molding by introduction of pressurized dry nitrogen at 75°C and 0.8 MPa for 120 seconds. Thus there was obtained a blown tube measuring 3.00 mm in outside diameter and 2.70 mm in inside diameter. The thus obtained blown tube was fabricated by means of ArF excimer laser (193 nm) to give the stent of the structure as shown in Fig. 8.

The stent of 3.00 mm in outside diameter and cut to a length of 10 mm was tested for radial force (rebound) exerted by it after compression by 1 mm in the same way as in Example 1. It was found that the radial force (rebound) was 123 gf.

The same procedure as mentioned above was repeated to produce the stent of the structure as shown in Fig. 1. It was found that this stent has a radial force (rebound) of 143 gf.

### Example 13

A mixture was prepared by dry blending from 100 pbw of lactic acid-trimethylene carbonate copolymer (made by Taki Chemical Co., Ltd., the same one as used in Example 11) and 1 pbw of 4-hydroxybenzoic acid (made by Sigma-Aldrich Corporation). The resulting mixture was formed into a tube, measuring 1.38 mm in outside diameter and 0.45 mm in inside diameter, by extrusion molding (with Labo Plastomill made by Toyo Seiki Seisaku-Sho, Ltd.). The resulting tube underwent blow molding by introduction of pressurized dry nitrogen at 55°C and 0.9 MPa for 120 seconds. Thus there was obtained a blown tube measuring 3.00 mm in outside diameter and 2.70 mm in inside diameter. The thus obtained blown tube was fabricated by means of ArF excimer laser (193 nm) to give the stent of the structure as shown in Fig. 8.

The stent of 3.00 mm in outside diameter and cut to a length of 10 mm was tested for radial force (rebound) exerted by it after compression by 1 mm in the same way as in Example 1. It was found that the radial force (rebound) was 140 gf.

The same procedure as mentioned above was repeated to produce the stent of the structure as shown in Fig. 1. It was found that this stent has a radial force (rebound) of 153 gf.

### Example 14

A mixture was prepared by dry blending from 100 pbw of lactic acid-trimethylene carbonate copolymer (made by Taki Chemical Co., Ltd., the same one as used in Example 11) and 1 pbw of 3-iodo-L-tyrosine (made by Tokyo Chemical Industry Co., Ltd.). The resulting mixture was formed into a tube, measuring 1.38 mm in outside diameter and 0.45 mm in inside diameter, by extrusion molding (with Labo Plastomill made by Toyo Seiki Seisaku-Sho, Ltd.). The resulting tube underwent blow molding by introduction of pressurized dry nitrogen at 65°C and 1.2 MPa for 120 seconds. Thus there was obtained a blown tube measuring 3.00 mm in outside diameter and 2.70 mm in inside diameter. The thus obtained blown tube was fabricated by means of ArF excimer laser (193 nm) to give the stent of the structure as shown in Fig. 8.

The stent of 3.00 mm in outside diameter and cut to a length of 10 mm was tested for radial force (rebound) exerted by it after compression by 1 mm in the same way as in Example 1. It was found that the radial force (rebound) was 139 gf. This stent was visible under X-ray radiography.

The same procedure as mentioned above was repeated to produce the stent of the structure as shown in Fig. 1. It was found that this stent has a radial force (rebound) of 146 gf. This stent was visible under X-ray radiography.

### Example 15

A mixture was prepared by dry blending from 100 pbw of lactic acid-trimethylene carbonate copolymer (made by Taki Chemical Co., Ltd., the same one as used in Example 11) and 1 pbw of 3,5-diiodo-salicylic acid (made by Sigma-Aldrich Corporation). The resulting mixture was formed into a tube, measuring 1.38 mm in outside diameter and 0.45 mm in inside diameter, by extrusion molding (with Labo Plastomill made by Toyo Seiki Seisaku-Sho, Ltd.). The resulting tube underwent blow molding by introduction of pressurized dry nitrogen at 60°C and 0.9 MPa for 120 seconds. Thus there was obtained a blown tube measuring 3.00 mm in outside diameter and 2.70 mm in inside diameter. The thus obtained blown tube was fabricated by means of ArF excimer laser (193 nm) to give the stent of the structure as shown in Fig. 8.

The stent of 3.00 mm in outside diameter and cut to a length of 10 mm was tested for radial force (rebound) exerted by it after compression by 1 mm in the same way as in Example 1. It was found that the radial force (rebound) was 136 gf. This stent was visible under X-ray radiography.

The same procedure as mentioned above was repeated to produce the stent of the structure as shown in Fig. 1. It was found that this stent has a radial force (rebound) of 146 gf. This stent was visible under X-ray radiography.

### Example 16

A mixture was prepared by dry blending from 100 pbw of lactic acid-trimethylene carbonate copolymer (made by Taki Chemical Co., Ltd., the same one as used in Example 11) and 7 pbw of mandelic acid (made by Sigma-Aldrich Corporation). The resulting mixture was formed into a tube, measuring 1.38 mm in outside diameter and 0.45 mm in inside diameter, by extrusion molding (with Labo Plastomill made by Toyo Seiki Seisaku-Sho, Ltd.). The resulting tube underwent blow molding by introduction of pressurized dry nitrogen at 60°C and 1.0 MPa for 120 seconds. Thus there was obtained a blown tube measuring 3.00 mm in outside diameter and 2.70 mm in inside diameter. The thus obtained blown tube was fabricated by means of ArF excimer laser (193 nm) to give the stent of the structure as shown in Fig. 8.

The stent of 3.00 mm in outside diameter and cut to a length of 10 mm was tested for radial force (rebound) exerted by it after compression by 1 mm in the same way as in Example 1. It was found that the radial force (rebound) was 123 gf.

The same procedure as mentioned above was repeated to produce the stent of the structure as shown in Fig. 1. It was found that this stent has a radial force (rebound) of 141 gf.

### Example 17

A mixture was prepared by dry blending from 100 pbw of lactic acid-trimethylene carbonate copolymer (made by Taki Chemical Co., Ltd., the same one as used in Example 11) and 7 pbw of 4-hydroxycinnamic acid (made by Tokyo Chemical Industry Co., Ltd.). The resulting mixture was formed into a tube, measuring 1.38 mm in outside diameter and 0.45 mm in inside diameter, by extrusion molding (with Labo Plastomill made by Toyo Seiki Seisaku-Sho, Ltd.). The resulting tube underwent blow molding by introduction of pressurized dry nitrogen at 75°C and 0.8 MPa for 120 seconds. Thus there was obtained a blown tube measuring 3.00 mm in outside diameter and 2.70 mm in inside diameter. The thus obtained blown tube was fabricated by means of ArF excimer laser (193 nm) to give the stent of the structure as shown in Fig. 8.

The stent of 3.00 mm in outside diameter and cut to a length of 10 mm was tested for radial force (rebound) exerted by it after compression by 1 mm in the same way as in Example 1. It was found that the radial force (rebound) was 120 gf.

The same procedure as mentioned above was repeated to produce the stent of the structure as shown in Fig. 1. It was found that this stent has a radial force (rebound) of 134 gf.

### Example 18

A mixture was prepared by dry blending from 100 pbw of lactic acid-trimethylene carbonate copolymer (made by Taki Chemical Co., Ltd., the same one as used in Example 11) and 7 pbw of 4-hydroxybenzoic acid (made by Sigma-Aldrich Corporation). The resulting mixture was formed into a tube, measuring 1.38 mm in outside diameter and 0.45 mm in inside diameter, by extrusion molding (with Labo Plastomill made by Toyo Seiki Seisaku-Sho, Ltd.). The resulting tube underwent blow molding by introduction of pressurized dry nitrogen at 55°C and 0.9 MPa for 120 seconds. Thus there was obtained a blown tube measuring 3.00 mm in outside diameter and 2.70 mm in inside diameter. The thus obtained blown tube was fabricated by means of ArF excimer laser (193 nm) to give the stent of the structure as shown in Fig. 8.

The stent of 3.00 mm in outside diameter and cut to a length of 10 mm was tested for radial force (rebound) exerted by it after compression by 1 mm in the same way as in Example 1. It was found that the radial force (rebound) was 136 gf.

The same procedure as mentioned above was repeated to produce the stent of the structure as shown in Fig. 1. It was found that this stent has a radial force (rebound) of 156 gf.

### Example 19

A mixture was prepared by dry blending from 100 pbw of lactic acid-trimethylene carbonate copolymer (made by Taki Chemical Co., Ltd., the same one as used in Example 11) and 7 pbw of 3-iodo-L-tyrosine (made by Tokyo Chemical Industry Co., Ltd.). The resulting mixture was formed into a tube, measuring 1.38 mm in outside diameter and 0.45 mm in inside diameter, by extrusion molding (with Labo Plastomill made by Toyo Seiki Seisaku-Sho, Ltd.). The resulting tube underwent blow molding by introduction of pressurized dry nitrogen at 65°C and 1.2 MPa for 120 seconds. Thus there was obtained a blown tube measuring 3.00 mm in outside diameter and 2.70 mm in inside diameter. The thus obtained blown tube was fabricated by means of ArF excimer laser (193 nm) to give the stent of the structure as shown in Fig. 8.

The stent of 3.00 mm in outside diameter and cut to a length of 10 mm was tested for radial force (rebound) exerted by it after compression by 1 mm in the same way as in Example 1. It was found that the radial force (rebound) was 146 gf. This stent was visible under X-ray radiography.

The same procedure as mentioned above was repeated to produce the stent of the structure as shown in Fig. 1. It was found that this stent has a radial force (rebound) of 159 gf. This stent was visible under X-ray radiography.

### Example 20

A mixture was prepared by dry blending from 100 pbw of lactic acid-trimethylene carbonate copolymer (made by Taki Chemical Co., Ltd., the same one as used in Example 11) and 7 pbw of 3,5-diiodo-salicylic acid (made by Sigma-Aldrich Corporation). The resulting mixture was formed into a tube, measuring 1.38 mm in outside diameter and 0.45 mm in inside diameter, by extrusion molding (with Labo Plastomill made by Toyo Seiki Seisaku-Sho, Ltd.). The resulting tube underwent blow molding by introduction of pressurized dry nitrogen at 60°C and 0.9 MPa for 120 seconds. Thus there was obtained a blown tube measuring 3.00 mm in outside diameter and 2.70 mm in inside diameter. The thus obtained blown tube was fabricated by means of ArF excimer laser (193 nm) to give the stent of the structure as shown in Fig. 8.

The stent of 3.00 mm in outside diameter and cut to a length of 10 mm was tested for radial force (rebound) exerted by it after compression by 1 mm in the same way as in Example 1. It was found that the radial force (rebound) was 141 gf. This stent was visible under X-ray radiography.

The same procedure as mentioned above was repeated to produce the stent of the structure as shown in Fig. 1. It was found that this stent has a radial force (rebound) of 156 gf. This stent was visible under X-ray radiography.

### Example 21

A mixture was prepared by dry blending from 100 pbw of polyglycolic acid having a weight-average molecular weight of 159,800 (made by DURECT Corporation) and 1 pbw of mandelic acid (made by Sigma-Aldrich Corporation). The resulting mixture was formed into a tube, measuring 1.38 mm in outside diameter and 0.45 mm in inside diameter, by extrusion molding (with Labo Plastomill made by Toyo Seiki Seisaku-Sho, Ltd.). The resulting tube underwent blow molding by introduction of pressurized dry nitrogen at 45°C and 2.0 MPa for 300 seconds. Thus there was obtained a blown tube measuring 3.00 mm in outside diameter and 2.70 mm in inside diameter. The thus obtained blown tube was fabricated by means of ArF excimer laser (193 nm) to give the stent of the structure as shown in Fig. 8.

The stent of 3.00 mm in outside diameter and cut to a length of 10 mm was tested for radial force (rebound) exerted by it after compression by 1 mm by means of an autograph (Model AG-IS, made by Shimadzu Seisakusho) It was found that the radial force (rebound) was 145 gf.

The same procedure as mentioned above was repeated to produce the stent of the structure as shown in Fig. 1. It was found that this stent has a radial force (rebound) of 173 gf.

### Example 22

A mixture was prepared by dry blending from 100 pbw of polyglycolic acid (made by DURECT Corporation, the same one as used in Example 21) and 1 pbw of 4-hydroxycinnamic acid (made by Tokyo Chemical Industry Co., Ltd.). The resulting mixture was formed into a tube, measuring 1.38 mm in outside diameter and 0.45 mm in inside diameter, by extrusion molding (with Labo Plastomill made by Toyo Seiki Seisaku-Sho, Ltd.). The resulting tube underwent blow molding by introduction of pressurized dry nitrogen at 45°C and 2.0 MPa for 300 seconds. Thus there was obtained a blown tube measuring 3.00 mm in outside diameter and 2.70 mm in inside diameter. The thus obtained blown tube was fabricated by means of ArF excimer laser (193 nm) to give the stent of the structure as shown in Fig. 8.

The stent of 3.00 mm in outside diameter and cut to a length of 10 mm was tested for radial force (rebound) exerted by it after compression by 1 mm by means of an autograph (Model AG-IS, made by Shimadzu Seisakusho). It was found that the radial force (rebound) was 138 gf.

The same procedure as mentioned above was repeated to produce the stent of the structure as shown in Fig. 1. It was found that this stent has a radial force (rebound) of 162 gf.

### Example 23

A mixture was prepared by dry blending from 100 pbw of polyglycolic acid (made by DURECT Corporation, the same one as used in Example 21) and 1 pbw of 4-hydroxybenzoic acid (made by Sigma-Aldrich Corporation). The resulting mixture was formed into a tube, measuring 1.38 mm in outside diameter and 0.45 mm in inside diameter, by extrusion molding (with Labo Plastomill made by Toyo Seiki Seisaku-Sho, Ltd.). The resulting tube underwent blow molding by introduction of pressurized dry nitrogen at 45°C and 2.0 MPa for 300 seconds. Thus there was obtained a blown tube measuring 3.00 mm in outside diameter and 2.70 mm in inside diameter. The thus obtained blown tube was fabricated by means of ArF excimer laser (193 nm) to give the stent of the structure as shown in Fig. 8.

The stent of 3.00 mm in outside diameter and cut to a length of 10 mm was tested for radial force (rebound) exerted by it after compression by 1 mm by means of an autograph (Model AG-IS, made by Shimadzu Seisakusho). It was found that the radial force (rebound) was 160 gf.

The same procedure as mentioned above was repeated to produce the stent of the structure as shown in Fig. 1. It was found that this stent has a radial force (rebound) of 186 gf.

### Example 24

A mixture was prepared by dry blending from 100 pbw of polyglycolic acid (made by DURECT Corporation, the same one as used in Example 21) and 1 pbw of 3-iodo-L-tyrosine (made by Tokyo Chemical Industry Co., Ltd.). The resulting mixture was formed into a tube, measuring 1.38 mm in outside diameter and 0.45 mm in inside diameter, by extrusion molding (with Labo Plastomill made by Toyo Seiki Seisaku-Sho, Ltd.). The resulting tube underwent blow molding by introduction of pressurized dry nitrogen at 45°C and 2.0 MPa for 300 seconds. Thus there was obtained a blown tube measuring 3.00 mm in outside diameter and 2.70 mm in inside diameter. The thus obtained blown tube was fabricated by means of ArF excimer laser (193 nm) to give the stent of the structure as shown in Fig. 8.

The stent of 3.00 mm in outside diameter and cut to a length of 10 mm was tested for radial force (rebound) exerted by it after compression by 1 mm by means of an autograph (Model AG-IS, made by Shimadzu Seisakusho). It was found that the radial force (rebound) was 171 gf. This stent was visible under X-ray radiography.

The same procedure as mentioned above was repeated to produce the stent of the structure as shown in Fig. 1. It was found that this stent has a radial force (rebound) of 196 gf. This stent was visible under X-ray radiography.

### Example 25

A mixture was prepared by dry blending from 100 pbw of polyglycolic acid (made by DURECT Corporation, the same one as used in Example 21) and 1 pbw of 3,5-diiodo-salicylic acid (made by Sigma-Aldrich Corporation). The resulting mixture was formed into a tube, measuring 1.38 mm in outside diameter and 0.45 mm in inside diameter, by extrusion molding (with Labo Plastomill made by Toyo Seiki Seisaku-Sho, Ltd.). The resulting tube underwent blow molding by introduction of pressurized dry nitrogen at 45°C and 2.0 MPa for 300 seconds. Thus there was obtained a blown tube measuring 3.00 mm in outside diameter and 2.70 mm in inside diameter. The thus obtained blown tube was fabricated by means of ArF excimer laser (193 nm) to give the stent of the structure as shown in Fig. 8.

The stent of 3.00 mm in outside diameter and cut to a length of 10 mm was tested for radial force (rebound) exerted by it after compression by 1 mm by means of an autograph (Model AG-IS, made by Shimadzu Seisakusho). It was found that the radial force (rebound) was 163 gf. This stent was visible under X-ray radiography.

The same procedure as mentioned above was repeated to produce the stent of the structure as shown in Fig. 1. It was found that this stent has a radial force (rebound) of 178 gf. This stent was visible under X-ray radiography.

### Example 26

A mixture was prepared by dry blending from 100 pbw of polyglycolic acid (made by DURECT Corporation, the same one as used in Example 21) and 7 pbw of mandelic acid (made by Sigma-Aldrich Corporation). The resulting mixture was formed into a tube, measuring 1.38 mm in outside diameter and 0.45 mm in inside diameter, by extrusion molding (with Labo Plastomill made by Toyo Seiki Seisaku-Sho, Ltd.). The resulting tube underwent blow molding by introduction of pressurized dry nitrogen at 45°C and 2.0 MPa for 300 seconds. Thus there was obtained a blown tube measuring 3.00 mm in outside diameter and 2.70 mm in inside diameter. The thus obtained blown tube was fabricated by means of ArF excimer laser (193 nm) to give the stent of the structure as shown in Fig. 8.

The stent of 3.00 mm in outside diameter and cut to a length of 10 mm was tested for radial force (rebound) exerted by it after compression by 1 mm by means of an autograph (Model AG-IS, made by Shimadzu Seisakusho). It was found that the radial force (rebound) was 149 gf.

The same procedure as mentioned above was repeated to produce the stent of the structure as shown in Fig. 1. It was found that this stent has a radial force (rebound) of 171 gf.

### Example 27

A mixture was prepared by dry blending from 100 pbw of polyglycolic acid (made by DURECT Corporation, the same one as used in Example 21) and 7 pbw of 4-hydroxycinnamic acid (made by Tokyo Chemical Industry Co., Ltd.). The resulting mixture was formed into a tube, measuring 1.38 mm in outside diameter and 0.45 mm in inside diameter, by extrusion molding (with Labo Plastomill made by Toyo Seiki Seisaku-Sho, Ltd.). The resulting tube underwent blow molding by introduction of pressurized dry nitrogen at 45°C and 2.0 MPa for 300 seconds. Thus there was obtained a blown tube measuring 3.00 mm in outside diameter and 2.70 mm in inside diameter. The thus obtained blown tube was fabricated by means of ArF excimer laser (193 nm) to give the stent of the structure as shown in Fig. 8.

The stent of 3.00 mm in outside diameter and cut to a length of 10 mm was tested for radial force (rebound) exerted by it after compression by 1 mm by means of an autograph (Model AG-IS, made by Shimadzu Seisakusho). It was found that the radial force (rebound) was 146 gf.

The same procedure as mentioned above was repeated to produce the stent of the structure as shown in Fig. 1. It was found that this stent has a radial force (rebound) of 170 gf.

### Example 28

A mixture was prepared by dry blending from 100 pbw of polyglycolic acid (made by DURECT Corporation, the same one as used in Example 21) and 7 pbw of 4-hydroxybenzoic acid (made by Sigma-Aldrich Corporation). The resulting mixture was formed into a tube, measuring 1.38 mm in outside diameter and 0.45 mm in inside diameter, by extrusion molding (with Labo Plastomill made by Toyo Seiki Seisaku-Sho, Ltd.). The resulting tube underwent blow molding by introduction of pressurized dry nitrogen at 45°C and 2.0 MPa for 300 seconds. Thus there was obtained a blown tube measuring 3.00 mm in outside diameter and 2.70 mm in inside diameter. The thus obtained blown tube was fabricated by means of ArF excimer laser (193 nm) to give the stent of the structure as shown in Fig. 8.

The stent of 3.00 mm in outside diameter and cut to a length of 10 mm was tested for radial force (rebound) exerted by it after compression by 1 mm by means of an autograph (Model AG-IS, made by Shimadzu Seisakusho). It was found that the radial force (rebound) was 155 gf.

The same procedure as mentioned above was repeated to produce the stent of the structure as shown in Fig. 1. It was found that this stent has a radial force (rebound) of 184 gf.

### Example 29

A mixture was prepared by dry blending from 100 pbw of polyglycolic acid (made by DURECT Corporation, the same one as used in Example 21) and 7 pbw of 3-iodo-L-tyrosine (made by Tokyo Chemical Industry Co., Ltd.). The resulting mixture was formed into a tube, measuring 1.38 mm in outside diameter and 0.45 mm in inside diameter, by extrusion molding (with Labo Plastomill made by Toyo Seiki Selsaku-Sho, Ltd.). The resulting tube underwent blow molding by introduction of pressurized dry nitrogen at 45°C and 2.0 MPa for 300 seconds. Thus there was obtained a blown tube measuring 3.00 mm in outside diameter and 2.70 mm in inside diameter. The thus obtained blown tube was fabricated by means of ArF excimer laser (193 nm) to give the stent of the structure as shown in Fig. 8.

The stent of 3.00 mm in outside diameter and cut to a length of 10 mm was tested for radial force (rebound) exerted by it after compression by 1 mm by means of an autograph (Model AG-IS, made by Shimadzu Seisakusho). It was found that the radial force (rebound) was 167 gf. This stent was visible under X-ray radiography.

The same procedure as mentioned above was repeated to produce the stent of the structure as shown in Fig. 1. It was found that this stent has a radial force (rebound) of 186 gf. This stent was visible under X-ray radiography.

### Example 30

A mixture was prepared by dry blending from 100 pbw of polyglycolic acid (made by DURECT Corporation, the same one as used in Example 21) and 7 pbw of 3,5-diiodo-salicylic acid (made by Sigma-Aldrich Corporation). The resulting mixture was formed into a tube, measuring 1.38 mm in outside diameter and 0.45 mm in inside diameter, by extrusion molding (with Labo Plastomill made by Toyo Seiki Seisaku-Sho, Ltd.). The resulting tube underwent blow molding by introduction of pressurized dry nitrogen at 45°C and 2.0 MPa for 300 seconds. Thus there was obtained a blown tube measuring 3.00 mm in outside diameter and 2.70 mm in inside diameter. The thus obtained blown tube was fabricated by means of ArF excimer laser (193 nm) to give the stent of the structure as shown in Fig. 8.

The stent of 3.00 mm in outside diameter and cut to a length of 10 mm was tested for radial force (rebound) exerted by it after compression by 1 mm by means of an autograph (Model AG-IS, made by Shimadzu Seisakusho). It was found that the radial force (rebound) was 163 gf. This stent was visible under X-ray radiography.

The same procedure as mentioned above was repeated to produce the stent of the structure as shown in Fig. 1. It was found that this stent has a radial force (rebound) of 177 gf. This stent was visible under X-ray radiography.

### Comparative Example 1

Polylactic acid (made by DURECT Corporation, the same one as used in Example 1) was formed into a tube, measuring 1.38 mm in outside diameter and 0.45 mm in inside diameter, by extrusion molding (with Labo Plastomill made by Toyo Seiki Seisaku-Sho, Ltd.). The resulting tube underwent blow molding by introduction of pressurized dry nitrogen at 90°C and 0.3 MPa for 120 seconds. Thus there was obtained a blown tube measuring 3.00 mm in outside diameter and 2.70 mm in inside diameter. The thus obtained blown tube was fabricated by means of ArF excimer laser (193 nm) to give the stent of the structure as shown in Fig. 8.

The stent of 3.00 mm in outside diameter and cut to a length of 10 mm was tested for radial force (rebound) exerted by it after compression by 1 mm in the same way as in Example 1. It was found that the radial force (rebound) was 92 gf.

The same procedure as mentioned above was repeated to produce the stent of the structure as shown in Fig. 1. It was found that this stent has a radial force (rebound) of 96 gf.

### Comparative Example 2

Lactic acid-trimethylene carbonate copolymer (made by Taki Chemical Co., Ltd., the same one as used in Example 11) was formed into a tube, measuring 1.38 mm in outside diameter and 0.45 mm in inside diameter, by extrusion molding (with Labo Plastomill made by Toyo Seiki Seisaku-Sho, Ltd.). The resulting tube underwent blow molding by introduction of pressurized dry nitrogen at 60°C and 1.0 MPa for 120 seconds. Thus there was obtained a blown tube measuring 3.00 mm in outside diameter and 2.70 mm in inside diameter. The thus obtained blown tube was fabricated by means of ArF excimer laser (193 nm) to give the stent of the structure as shown in Fig. 8.

The stent of 3.00 mm in outside diameter and cut to a length of 10 mm was tested for radial force (rebound) exerted by it after compression by 1 mm in the same way as in Example 1. It was found that the radial force (rebound) was 83 gf.

The same procedure as mentioned above was repeated to produce the stent of the structure as shown in Fig. 1. It was found that this stent has a radial force (rebound) of 89 gf.

### Comparative Example 3

Polyglycolic acid (made by DURECT Corporation, the same one as used in Example 21) was formed into a tube, measuring 1.38 mm in outside diameter and 0.45 mm in inside diameter, by extrusion molding (with Labo Plastomill made by Toyo Seiki Seisaku-Sho, Ltd.). The resulting tube underwent blow molding by introduction of pressurized dry nitrogen at 45°C and 2.0 MPa for 300 seconds. Thus there was obtained a blown tube measuring 3.00 mm in outside diameter and 2.70 mm in inside diameter. The thus obtained blown tube was fabricated by means of ArF excimer laser (193 nm) to give the stent of the structure as shown in Fig. 8.

The stent of 3.00 mm in outside diameter and cut to a length of 10 mm was tested for radial force (rebound) exerted by it after compression by 1 mm in the same way as in Example 1. It was found that the radial force (rebound) was 89 gf.

The same procedure as mentioned above was repeated to produce the stent of the structure as shown in Fig. 1. It was found that this stent has a radial force (rebound) of 93 gf.

### Comparative Example 4

Polycaprolactone having a weight-average molecular weight of 115,000 (made by DURECT Corporation) was formed into a tube, measuring 1.38 mm in outside diameter and 0.45 mm in inside diameter, by extrusion molding (with Labo Plastomill made by Toyo Seiki Seisaku-Sho, Ltd.). The resulting tube underwent blow molding by introduction of pressurized dry nitrogen at 40°C and 0.1 MPa for 120 seconds. Thus there was obtained a blown tube measuring 3.00 mm in outside diameter and 2.70 mm in inside diameter. The thus obtained blown tube was fabricated by means of ArF excimer laser (193 nm) to give the stent of the structure as shown in Fig. 8.

The stent of 3.00 mm in outside diameter and cut to a length of 10 mm was tested for radial force (rebound) exerted by it after compression by 1 mm in the same way as in Example 1. It was found that the radial force (rebound) was 38 gf.

The same procedure as mentioned above was repeated to produce the stent of the structure as shown in Fig. 1. It was found that this stent has a radial force (rebound) of 42 gf.

### Comparative Example 5

A mixture was prepared by dry blending from 100 pbw of polylactic acid (made by DURECT Corporation, the same one as used in Example 1) and 1 pbw of succinic acid (made by Sigma-Aldrich Corporation). The resulting mixture was formed into a tube, measuring 1.38 mm in outside diameter and 0.45 mm in inside diameter, by extrusion molding (with Labo Plastomill made by Toyo Seiki Seisaku-Sho, Ltd.). The resulting tube underwent blow molding by introduction of pressurized dry nitrogen at 80°C and 0.4 MPa for 120 seconds. Thus there was obtained a blown tube measuring 3.00 mm in outside diameter and 2.70 mm in inside diameter. The thus obtained blown tube was fabricated by means of ArF excimer laser (193 nm) to give the stent of the structure as shown in Fig. 8.

The stent of 3.00 mm in outside diameter and cut to a length of 10 mm was tested for radial force (rebound) exerted by it after compression by 1 mm in the same way as in Example 1. It was found that the radial force (rebound) was 83 gf.

### Comparative Example 6

A mixture was prepared by dry blending from 100 pbw of polylactic acid (made by DURECT Corporation, the same one as used in Example 1) and 1 pbw of adipic acid (made by Sigma-Aldrich Corporation). The resulting mixture was formed into a tube, measuring 1.38 mm in outside diameter and 0.45 mm in inside diameter, by extrusion molding (with Labo Plastomill made by Toyo Seiki Seisaku-Sho, Ltd.). The resulting tube underwent blow molding by introduction of pressurized dry nitrogen at 80°C and 0.4 MPa for 120 seconds. Thus there was obtained a blown tube measuring 3.00 mm in outside diameter and 2.70 mm in inside diameter. The thus obtained blown tube was fabricated by means of ArF excimer laser (193 nm) to give the stent of the structure as shown in Fig. 8.

The stent of 3.00 mm in outside diameter and cut to a length of 10 mm was tested for radial force (rebound) exerted by it after compression by 1 mm in the same way as in Example 1. It was found that the radial force (rebound) was 86 gf.

### Comparative Example 7

A stent of stainless steel (SUS 316L) was prepared which has the structure as shown in Fig. 1 and measures 3.00 mm in outside diameter and 10 mm in length. This stent was tested for radial force (rebound) exerted by it after compression by 1 mm in the same way as in Example 1. It was found that the radial force (rebound) was 196 gf.

Tables 1 to 3 below summarize the results of Examples 1 to 10 and Comparative Examples 1 to 7, the results of Examples 11 to 20 and Comparative Examples 1 to 7, and the results of Examples 21 to 30 and Comparative Examples 1 to 7, respectively.

**Table 1**

| | Bioabsorbable aliphatic polyester | | Aromatic compound | | Radial force of stent of Fig. 8 (gf) | Radial force of stent of Fig. 1 (gf) |
|---|---|---|---|---|---|---|
| | Name | Mw | Name | Mixing ratio *¹ (by mass) | | |
| Example 1 | Polylactic acid | 123,000 | Mandelic acid | 1 | 139 | 164 |
| Example 2 | Polylactic acid | 123,000 | 4-hydroxycinnamic acid | 1 | 131 | 155 |
| Example 3 | Polylactic acid | 123,000 | 4-hydroxybenzoic acid | 1 | 150 | 173 |
| Example 4 | Polylactic acid | 123,000 | 3-iodo-L-tyrosine | 1 | 159 | 183 |
| Example 5 | Polylactic acid | 123,000 | 3,5-diiodo-salicylic acid | 1 | 150 | 163 |
| Example 6 | Polylactic acid | 123,000 | Mandelic acid | 7 | 139 | 164 |
| Example 7 | Polylactic acid | 123,000 | 4-hydroxycinnamic acid | 7 | 131 | 155 |
| Example 8 | Polylactic acid | 123,000 | 4-hydroxybenzoic acid | 7 | 150 | 173 |
| Example 9 | Polylactic acid | 123,000 | 3-iodo-L-tyrosine | 7 | 159 | 183 |
| Example 10 | Polylactic acid | 123,000 | 3,5-diiodo-salicylic acid | 7 | 150 | 163 |
| Comp. Ex. 1 | Polylactic acid | 123,000 | - | - | 92 | 96 |
| Comp. Ex. 2 | Lactic acid-trimethylene carbonate copolymer | 100,000 | - | - | 83 | 89 |
| Comp. Ex. 3 | Polyglycolic acid | 159,800 | - | - | 89 | 93 |
| Comp. Ex. 4 | Polycaprolactone | 115,000 | - | - | 38 | 42 |
| Comp. Ex. 5 | Polylactic acid | 123,000 | Scuccinic acid *² | 1 | 83 | N.D. |
| Comp. Ex. 6 | Polylactic acid | 123,000 | Adipic acid *² | 1 | 86 | N.D. |
| Comp. Ex. 7 | Stent of stainless steel | | | | N.D. | 196 |

| | | | | | | |
|---|---|---|---|---|---|---|
| Mw = Weight-average molecular weight, N.D. = not determined, Comp. Ex. = Comparative Example *1: Mixing ratio (by mass) of aromatic compound to 100 pbw of bioabsorbable aliphatic polyester *2: Succinic acid and adipic acid are aliphatic compounds. | | | | | | |

**Table 2**

| | Bioabsorbable aliphatic polyester | | Aromatic compound | | Radial force of stent of Fig. 8 (gf) | Radial force of stent of Fig. 1 (gf) |
|---|---|---|---|---|---|---|
| | Name | Mw | Name | Mixing ratio *¹ (by mass) | | |
| Example 11 | Lactic acid-trimethylene carbonate copolymer | 100,000 | Mandelic acid | 1 | 131 | 158 |
| Example 12 | Lactic acid-trimethylene carbonate copolymer | 100,000 | 4-hydroxycinnamic acid | 1 | 123 | 143 |
| Example 13 | Lactic acid-trimethylene carbonate copolymer | 100,000 | 4-hydroxybenzoic acid | 1 | 140 | 153 |
| Example 14 | Lactic acid-trimethylene carbonate copolymer | 100,000 | 3-iodo-L-tyrosine | 1 | 139 | 146 |
| Example 15 | Lactic acid-trimethylene carbonate copolymer | 100,000 | 3,5-diiodo-salicylic acid | 1 | 136 | 146 |
| Example 16 | Lactic acid-trimethylene carbonate copolymer | 100,000 | Mandelic acid | 7 | 123 | 141 |
| Example 17 | Lactic acid-trimethylene carbonate copolymer | 100,000 | 4-hydroxycinnamic acid | 7 | 120 | 134 |
| Example 18 | Lactic acid-trimethylene carbonate copolymer | 100,000 | 4-hydroxybenzoic acid | 7 | 136 | 156 |
| Example 19 | Lactic acid-trimethylene carbonate copolymer | 100,000 | 3-iodo-L-tyrosine | 7 | 146 | 159 |
| Example 20 | Lactic acid-trimethylene carbonate copolymer | 100,000 | 3,5-diiodo-salicylic acid | 7 | 141 | 156 |
| Comp. Ex. 1 | Polylactic acid | 123,000 | - | - | 92 | 96 |
| Comp. Ex. 2 | Lactic acid-trimethylene carbonate copolymer | 100,000 | - | - | 83 | 89 |
| Comp. Ex. 3 | Polyglycolic acid | 159,800 | - | - | 89 | 93 |
| Comp. Ex. 4 | Polycaprolactone | 115,000 | - | - | 38 | 42 |
| Comp. Ex. 5 | Polylactic acid | 123,000 | Scuccinic acid *² | 1 | 83 | N.D. |
| Comp. Ex. 6 | Polylactic acid | 123,000 | Adipic acid *² | 1 | 86 | N.D. |
| Comp. Ex. 7 | Stent of stainless steel | | | | N.D. | 196 |

| | | | | | | |
|---|---|---|---|---|---|---|
| Mw = Weight-average molecular weight, N.D. = not determined, Comp. Ex. = Comparative Example *1: Mixing ratio (by mass) of aromatic compound to 100 pbw of bioabsorbable aliphatic polyester *2: Scuccinic acid and adipic acid are aliphatic compounds. | | | | | | |

**Table 3**

| | Bioabsorbable aliphatic polyester | | Aromatic compound | | Radial force of stent of Fig. 8 (gf) | Radial force of stent of Fig. 1 (gf) |
|---|---|---|---|---|---|---|
| | Name | Mw | Name | Mixing ratio *¹ (by mass) | | |
| Example 21 | Polyglycolic acid | 159,800 | Mandelic acid | 1 | 145 | 173 |
| Example 22 | Polyglycolic acid | 159,800 | 4-hydroxcinnamic acid | 1 | 138 | 162 |
| Example 23 | Polyglycolic acid | 159,800 | 4-hydroxybenzoic acid | 1 | 160 | 186 |
| Example 24 | Polyglycolic acid | 159,800 | 3-iodo-L-tyrosine | 1 | 171 | 196 |
| Example 25 | Polyglycolic acid | 159,800 | 3,5-diiodo-salicylic acid | 1 | 163 | 178 |
| Example 26 | Polyglycolic acid | 159,800 | Mandelic acid | 7 | 149 | 171 |
| Example 27 | Polyglycolic acid | 159,800 | 4-hydroxycinnamic acid | 7 | 146 | 170 |
| Example 28 | Polyglycolic acid | 159,800 | 4-hydroxybenzoic acid | 7 | 155 | 184 |
| Example 29 | Polyglyoolic acid | 159,800 | 3-iodo-L-tyrosine | 7 | 167 | 186 |
| Example 30 | Polyglycolic acid | 159,800 | 3,5-diiodo-salicylic-acid | 7 | 163 | 177 |
| Comp. Ex. 1 | Polylactic acid | 123,000 | - | - | 92 | 96 |
| Comp. Ex. 2 | Lactic acid-trimethylene carbonate copolymer | 100,000 | - | - | 83 | 89 |
| Comp. Ex. 3 | Polyglycolic acid | 159,800 | - | - | 89 | 93 |
| Comp. Ex. 4 | Polycaprolactone | 115,000 | - | - | 38 | 42 |
| Comp. Ex. 5 | Polylactic acid | 123,000 | Scuccinic acid *² | 1 | 8'3 | N.D. |
| Comp. Ex. 6 | Polylactic acid | 123,000 | Adipic acid *² | 1 | 86 | N.D. |
| Comp. Ex. 7 | Stent of stainless steel | | | | N.D. | 196 |

| | | | | | | |
|---|---|---|---|---|---|---|
| Mw = Weight-average molecular weight, N.D. = not determined, Comp. Ex. = Comparative Example *1: Mixing ratio (by mass) of aromatic compound to 100 pbw of bioabsorbable aliphatic polyester *2: Succinic acid and adipic acid are aliphatic compounds. | | | | | | |

The present application is based on the Japanese Patent Application No. 2010-021542 filed February 2, 2010.

## Claims

1. A bioabsorbable stent formed from a mixture composed of a bioabsorbable aliphatic polyester and an aromatic compound having one or more aromatic rings selected from the group including 2-hydroxybenzoic acid, 3-hydroxybenzoic acid, 4-hydroxybenzoic acid, 2,3-dihydroxybenzoic acid, 2,4-dihydroxybenzoic acid, 2,5-dihydroxybenzoic acid, 2,6-dihydroxybenzoic acid, 3,5-dihydroxybenzoic acid, 2-hydroxycinnamic acid, 3-hydroxycinnamic acid, 4-hydroxycinnamic acid, 4-hydroxy-2-methoxycinnamic acid, 4-hydroxy-3-methoxycinnamic acid, 3,4-dihydroxycinnamic acid, mandelic acid, and tyrosine and iodide compounds thereof.

2. The bioabsorbable stent as defined in Claim 1, wherein the bioabsorbable aliphatic polyester and the aromatic compound are mixed in a ratio (by mass) ranging from 100:0.1 to 100:9.

3. The bioabsorbable stent as defined in any one of Claims 1 to 2, wherein the bioabsorbable aliphatic polyester is at least one selected from the group including polylactic acid, polyglycolic acid, copolymer of lactic acid and glycolic acid, polycaprolactone, copolymer of lactic acid and caprolactone, copolymer of glycolic acid and caprolactone, polytrimethylene carbonate, copolymer of lactic acid and trimethylene carbonate, copolymer of glycolic acid and trimethylene carbonate, polydioxanone, polyethylene succinate, polybutylene succinate, and polybutylene succinate-adipate.

4. The bioabsorbable stent as defined in any one of Claims 1 to 3, which is formed by blow molding from a mixture composed of a bioabsorbable aliphatic polyester and an aromatic compound having one or more aromatic rings.

## Patentansprüche

1. Biologisch abbaubarer Stent, der aus einer Mischung gebildet ist, die sich aus einem biologisch abbaubaren aliphatischen Polyester und einer aromatischen Verbindung zusammensetzt, die ein oder mehrere aromatische Ringe aufweist, die aus der aus 2-Hydroxybenzoesäure, 3-Hydroxybenzoesäure, 4-Hydroxybenzoesäure, 2,3-Dihydroxybenzoesäure, 2,4-Dihydroxybenzoesäure, 2,5-Dihydroxybenzoesäure, 2,6-Dihydroxybenzoesäure, 3,5-Dihydroxybenzoesäure, 2-Hydroxyzimtsäure, 3-Hydroxyzimtsäure, 4-Hydroxyzimtsäure, 4-Hydroxy-2-methoxyzimtsäure, 4-Hydroxy-3-methoxyzimtsäure, 3,4-Dihydroxyzimtsäure, Mandelsäure sowie Tyrosin und Iodidverbindungen davon bestehenden Gruppe ausgewählt sind.

2. Biologisch abbaubarer Stent nach Anspruch 1, wobei der biologisch abbaubare aliphatische Polyester und die aromatische Verbindung in einem Verhältnis (massebezogen) im Bereich von 100:0,1 bis 100:9 gemischt sind.

3. Biologisch abbaubarer Stent nach einem der Ansprüche 1 bis 2, wobei der biologisch abbaubare aliphatische Polyester mindestens einer ist, der aus der aus Polymilchsäure, Polyglycolsäure, Copolymer von Milchsäure und Glycolsäure, Polycaprolacton, Copolymer von Milchsäure und Caprolacton, Copolymer von Glycolsäure und Caprolacton, Polytrimethylencarbonat, Copolymer von Milchsäure und Trimethylencarbonat, Copolymer von Glycolsäure und Trimethylencarbonat, Polydioxanon, Polyethylensuccinat, Polybutylensuccinat und Polybutylensuccinatadipat bestehenden Gruppe ausgewählt ist.

4. Biologisch abbaubarer Stent nach einem der Ansprüche 1 bis 3, der gebildet ist durch Blasformen aus einer Mischung, die sich aus einem biologisch abbaubaren aliphatischen Polyester und einer aromatischen Verbindung mit einem oder mehreren aromatischen Ringen zusammensetzt.

## Revendications

1. Endoprothèse biorésorbable, formée d'un mélange composé d'un polyester aliphatique bioabsorbable et d'un composé aromatique comportant un ou plusieurs cycle(s) aromatique(s), choisi dans l'ensemble comprenant les suivants : acide 2-hydroxy-benzoïque, acide 3-hydroxy-benzoïque, acide 4-hydroxy-benzoïque, acide 2,3-dihydroxy-benzoïque, acide 2,4-dihydroxy-benzoïque, acide 2,5-dihydroxy-benzoïque, acide 2,6-dihydroxy-benzoïque, acide 3,5-dihydroxy-benzoïque, acide 2-hydroxy-cinnamique, acide 3-hydroxy-cinnamique, acide 4-hydroxy-cinnamique, acide 4-hydroxy-2-méthoxy-cinnamique, acide 4-hydroxy-3-méthoxy-cinnamique, acide 3,4-dihydroxy-cinnamique, acide mandélique et tyrosine, ainsi que leurs dérivés iodés.

2. Endoprothèse biorésorbable conforme à la revendication 1, dans laquelle le polyester aliphatique bioabsorbable et le composé aromatique sont mélangés en un rapport massique valant de 100/0,1 à 100/9.

3. Endoprothèse biorésorbable conforme à l'une des revendications 1 à 2, dans laquelle le polyester aliphatique bioabsorbable est au nombre d'au moins un, choisi(s) dans l'ensemble comprenant les suivants : poly(acide lactique), poly(acide glycolique), copolymère d'acide lactique et d'acide glycolique, polycaprolactone, copolymère d'acide lactique et de caprolactone, copolymère d'acide glycolique et de caprolactone, poly(carbonate de triméthylène), copolymère d'acide lactique et de carbonate de triméthylène, copolymère d'acide glycolique et de carbonate de triméthylène, polydioxanone, poly(succinate d'éthylène), poly(succinate de butylène), et poly(succinate/adipate de butylène).

4. Endoprothèse biorésorbable conforme à l'une des revendications 1 à 3, qui est formée, par moulage par soufflage, à partir d'un mélange composé d'un polyester aliphatique bioabsorbable et d'un composé aromatique comportant un ou plusieurs cycle(s) aromatique(s).
